# EUROPEAN PATENT SPECIFICATION

(11) **EP 2 793 948 B1**
(45) Date of publication and mention of the grant of the patent: **23.03.2022**
(21) Application number: 12812433.6
(22) Date of filing: 04.12.2012
(51) Int. Cl.: A61K 47/60, A61K 38/24, A61P 35/00

(54) **THYROID STIMULATING HORMONE COMPOSITIONS**
SCHILDDRÜSENSTIMULIERENDE HORMONZUSAMMENSETZUNGEN
COMPOSITIONS DE THYRÉOSTIMULINE

(30) Priority: 19.12.2011 US 201161577412 P
(43) Date of publication of application: 29.10.2014
(73) Proprietor: Genzyme Corporation, Cambridge, MA 02142 (US)
(72) Inventor: PAN, Clark, Bridgewater, New Jersey 08807 (US); PARK, Sunghae, Bridgewater, New Jersey 08807 (US); QIU, Huawei, Bridgewater, New Jersey 08807 (US)
(74) Representative: Lavoix
(86) International application number: PCT/US2012/067705
(87) International publication number: WO 2013/095905

(56) References cited:
- WO-A1-2004/009672
- WO-A1-2008/036271
- WO-A1-2012/073125
- WO-A2-2005/056046
- US-A- 4 179 337

## Description

### BACKGROUND OF THE INVENTION

Thyroid cancer is a collection of diseases in which there is uncontrolled growth of cells derived from the thyroid. Thyroid cancer commonly has been classified as differentiated thyroid cancer, including papillary, follicular and Hurthle cell cancer, and other thyroid cancers, including medullary and anaplastic cancer. Over time, some differentiated thyroid cancers become less well differentiated, and may be classified as de-differentiated or poorly-differentiated cancer. Administration of Thyroid Stimulating Hormone (TSH) to patients can play a role in the diagnostic or therapeutic approach for various thyroid diseases, including goiter and thyroid cancer. For these diseases, the pharmacokinetic profile of the administered TSH may be important for the optimal success of the diagnostic or therapeutic procedures.

Recombinant human TSH (rhTSH), marketed as THYROGEN^{®} Thyroid Stimulating Hormone (Genzyme Corp., NDA 2-898), is dosed in multiple injections on consecutive days, followed by RAI dosing and blood draw for tumor diagnostics on day 3 and 5. This strict dosing regimen has been necessitated by the relatively short duration of action of TSH, which also results in reduced efficacy and side effects. A TSH composition with prolonged duration of action will likely improve treatment and detection of various thyroid diseases and reduce side effects.

WO 2005/056046 refers to methods for treating inflammation using THS, wherein a polyalkyl oxide polymer is attached to its N-terminus.

Thus, a need exists for improved TSH-containing compositions that optimize the pharmacokinetics of TSH release and reduce the need of multiple injections.

### SUMMARY OF THE INVENTION

The present invention relates to compositions of recombinant Thyroid Stimulating Hormone (rhTSH) conjugated with a (one or more) polyalkylene glycol polymer, such as polyethylene glycol (PEG), that prolongs the duration of rhTSH action *in vivo,* according to claims 1 and 7. Additionally, the invention relates to methods for producing a rhTHS conjugated to at least one polyalkylene glycol polymer according to claims 12 and 13. The rhTSH of the present invention described herein can be used for treatment of patients in need thereof with thyroid conditions according to claim 18.

The invention pertains to compositions comprising human rhTSH, wherein at least one polyalkylene glycol polymer is attached to a carbohydrate site of the rhTSH at a) a sialic acid group selected from the group consisting of ASN52 of the rhTSH α subunit, ASN78 of the rhTSH α subunit, and ASN23 of the rhTSH β subunit or a combination thereof; or at b)a galactose located at ASN52 of the rhTSH α subunit, ASN78 of the rhTSH α subunit, ASN23 of the rhTSH β subunit or a combination thereof. In certain aspects, the TSH of the compositions is isolated from a mammal, for example, a human, or the TSH is recombinant mammalian TSH, for example, recombinant human TSH (rhTSH).

In related aspects of the invention, the compositions of the invention exhibits an enhanced T4 response compared to a control.

In other related aspects of the invention, the polyalkylene glycol polymer attached to the carbohydrate site of TSH is polyethylene glycol (PEG). In particular embodiments, the PEG has an average molecular weight of between about 3,000 and about 100,000 Daltons. In other embodiments, one or more than one linear or branched PEG molecules is/are attached to TSH.

The invention further pertains to a composition comprising a mutated recombinant human Thyroid Stimulating Hormone (rhTSH) and at least one polyalkylene glycol polymer, wherein the mutated rhTSH comprises a rhTSH in which one or more amino acid residues has been substituted with a cysteine residue, wherein the amino acid residue that has been substituted with cysteine is located on the alpha subunit of rhTSH at an amino acid position selected from the group consisting of ASN66, THR69, and GLY22, and combinations thereof; or is located on the beta subunit of rhTSH at the amino acid position of VAL118; or any combination thereof; and wherein the polyalkylene glycol polymer is attached to the mutated rhTSH at the cysteine residue, and the mutated rhTSH is biologically active is described.

In certain aspects, the mutated TSH with the cysteine modification has attached polyethylene glycol (PEG) as the polyalkylene glycol polymer. In particular embodiments, the PEG has an average molecular weight of between about 3,000 and about 100,000 Daltons. In other embodiments, one or more than one linear or branched or combinations of PEG molecules is/are attached to TSH.

In accordance with other embodiments of the invention, pharmaceutical compositions comprising an effective therapeutic amount of a composition of the invention along with a pharmaceutically acceptable carrier are described.

These pharmaceutical compositions are used for treating a thyroid condition in a patient in need thereof, by administering to the patient an effective amount of the pharmaceutical compositions of the invention. In particular embodiments, the thyroid condition is thyroid cancer. In particular embodiments, the composition is delivered by intramuscular injection.

The invention also relates to methods of producing a PEGylated, biologically active recombinant human thyroid stimulating hormone (rhTSH) comprising attaching at least one polyalkylene glycol polymer to a carbohydrate site of a rhTSH at a) a sialic acid group selected from the group consisting of ASN52 of the rhTSH α subunit, ASN78 of the rhTSH α subunit, and ASN23 of the rhTSH β subunit or a combination thereof; or at b)a galactose located at ASN52 of the rhTSH α subunit, ASN78 of the rhTSH α subunit, ASN23 of the rhTSH β subunit or a combination thereof.

In another related aspect, the invention pertains to a method of producing a PEGylated, biologically active mutated recombinant human thyroid stimulating hormone (rhTSH) comprising (a) introducing one or more additional cysteine residues into the amino acid sequence of TSH, thereby producing a mutated TSH, wherein the cysteine residue replaces an endogenous amino acid residue located on the alpha subunit of rhTSH at an amino acid position selected from the group consisting of ASN66, THR69, and GLY22, and combinations thereof, or located on the beta subunit of rhTSH at the amino acid position of VAL118, and combinations thereof; and (b) attaching one or more polyalkylene glycol polymers to the one or more cysteine residues introduced in step a), thereby producing a biologically active mutated rhTSH conjugated to at least one polyalkylene glycol polymer. In particular embodiments, the cysteine residue replaces an endogenous amino acid residue in rhTSH.

The invention also relates to compositions for use in treating a thyroid condition in a subject in need thereof, comprising administering an effective amount of the PEGylated, biologically active recombinant human thyroid stimulating hormone (rhTSH) of the present invention.

### BRIEF DESCRIPTION OF THE DRAWINGS

The foregoing will be apparent from the following more particular description of example embodiments of the invention, as illustrated in the accompanying drawings in which like reference characters refer to the same parts throughout the different views. The drawings are not necessarily to scale, emphasis instead being placed upon illustrating embodiments of the present invention.
FIG. 1A - FIG. 1B are linear (FIG. 1A) and three-dimensional (FIG. 1B) schematics showing the structural modeling of the TSH subunits with PEGylation targets at N-termini, lysine amino acid residues and natural carbohydrate sites.

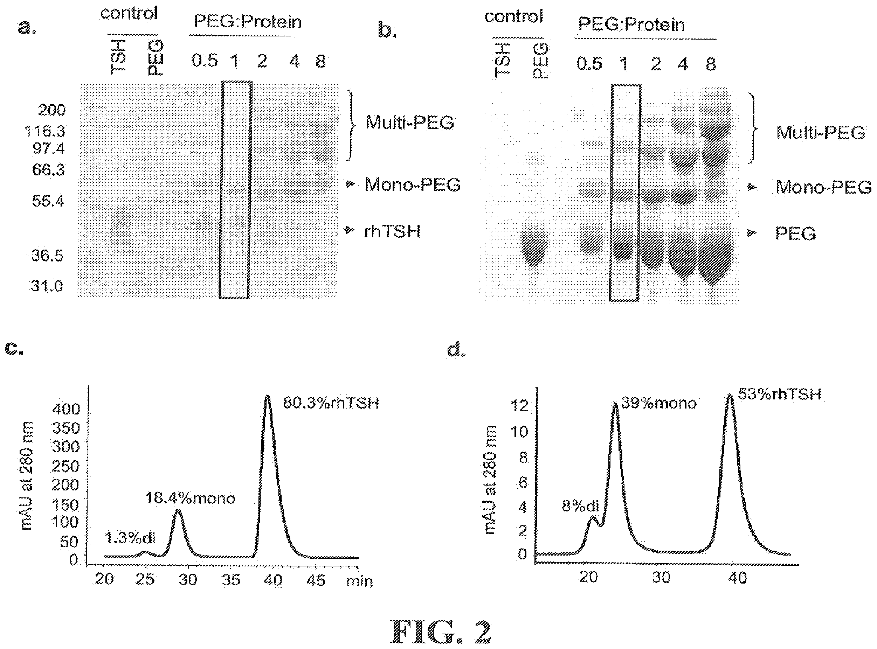

FIG. 2A - FIG. 2D are Lysine- and N-terminal PEGylation reactions. SDS-PAGE analysis of PEGylation reaction mixture of Lysine PEGylation with different PEG:protein ratio are shown in FIG. 2A (Coomassie blue stain) and FIG. 2B (PEG stain). Reaction mixture with 1:1 PEG:protein ratio is highlighted with boxes, which was further analyzed on a SEC-HPLC (FIG. 2C). FIG. 2D is a SEC-HPLC profile of an N-terminal PEGylation reaction at 2:1 PEG:protein ratio.
FIG. 3 is a schematic showing three different carbohydrate PEGylation pathways.
FIG. 4 is a structural model showing the beta-carbon positions of the selected mutants.
FIG. 5 is a silver-stained non-reducing SDS-PAGE to assess expression level and oligomerization of the mutants.
FIG. 6 is a gel showing the PEGylation of three cysteine site-directed mutants.

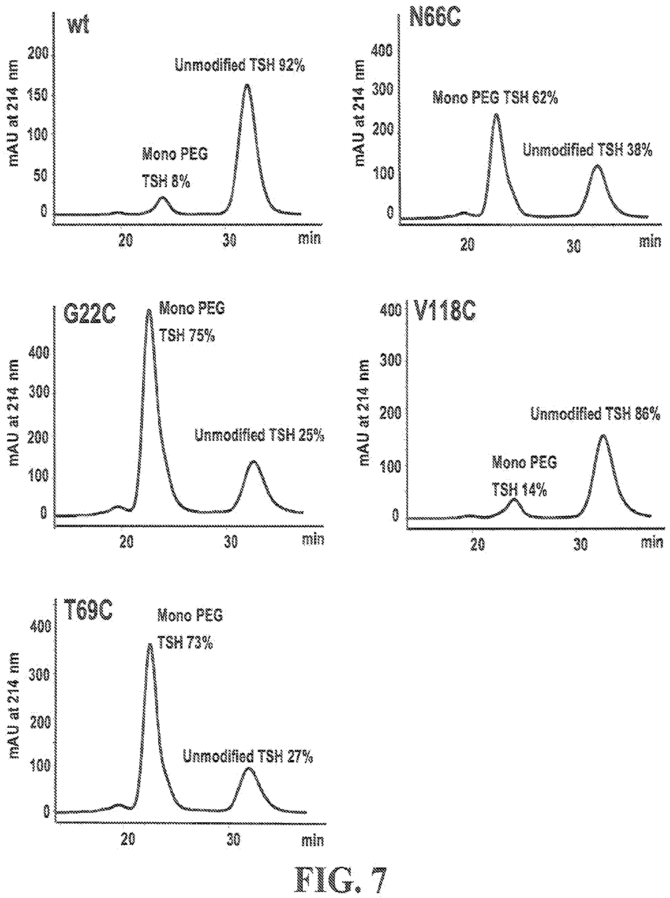

FIG. 7 is a series of chromatograms of the SEC-HPLC profiles of several PEGylation reactions of mutant TSH.
FIG. 8A - FIG. 8B are the optimization of αG22C TSH PEGylation conditions.
FIG. 9A - FIG. 9B are confirmation of subunit- and site-specific conjugation for G22C TSH
FIG. 10A - FIG. 10D are SDS-PAGE analysis of carbohydrate PEGylation reaction mixtures and purified carbohydrate PEGylated rhTSH conjugates. GAM(+) and GAM(-) reaction mixtures with varied (PEG:Protein) molar ratios are shown in FIG. 10A and FIG. 10B, respectively. Purified carbohydrate PEGylated rhTSH conjugates are shown in FIG. 10C (PEG staining) and FIG. 10D (Coomassie blue staining).
FIG. 11A - FIG. 11B are SEC-HPLC profiles of the 40kD SAM reaction mixture (FIG. 11A) and purified carbohydrate PEGylated rhTSH conjugates (FIG. 11B).
FIG. 12 is a tryptic peptide map of purified monoPEGylated 40kD SAM conjugate and the N-terminal sequencing result of the collected PEGylated tryptic fragments as explained in Example 10.
FIG. 13A - FIG. 13C are determination of the relative amount of PEGylation on each subunit of purified carbohydrate PEGylated rhTSH conjugates as explained in Example 11.
FIG. 14A - FIG. 14C are graphs showing the results of receptor binding assays of PEGylated SAM, GAM(+), GAM(-) with various sized PEG.
FIG. 15 is a graph of pharmacodynamic data of various PEGylated TSH showing the effects on T4 levels (µg/dL) over time relative to rhTSH control as explained in Example 13.
FIG. 16 is a graph showing the concentration of various PEGylated TSH in serum over time compared with control as explained in Example 13.
FIG. 17 is a graph showing the concentration of various PEGylated TSH in serum over time compared with control as explained in Example 14.
FIG. 18A - FIG.18D are graphs showing the concentration of T4 in serum for various PEGylated TSH over time compared with control as explained in Example 16.
FIG. 19A- FIG. 19C are graphs showing the concentration of T4 in serum for different doses of 40kD SAM over time compared with control as explained in Example 17.
FIG. 20A - FIG. 20B are graphs showing the concentration of T4 in serum for various PEGylated TSH over time compared with control as explained in Example 18.
FIG. 21A - FIG. 21F are graphs showing the concentration of T4 in serum for various PEGylated TSH over time compared with control as explained in Example 19.
FIG. 22 is a graph showing the concentration of T4 in serum for various PEGylated TSH over time compared with control as explained in Example 19.
FIG. 23 is a graph showing the concentration of T4 in serum for 10kD multiSAM and 40kD SAM over time as explained in Example 20.
FIG. 24 is a graph showing the concentration of T4 in serum for 40kD SAM and 40kD G22C over time compared with control as explained in Example 21.

### DETAILED DESCRIPTION OF THE INVENTION

THYROGEN^{®}, Thyroid Stimulating Hormone (Genzyme Corp., NDA 2-898) is recombinant human TSH (rhTSH) currently marketed for the diagnosis and/or treatment of thyroid cancer. It is sold as a lyophilized powder for reconstitution with water prior to intramuscular administration.

Such existing formulations of rhTSH have rigid dosing regimes, require multiple, injections, have limited pharmacokinetic profiles and produce side effects, such as nausea. These shortcomings are addressed by the rhTSH compositions and mutated rhTSH compositions of the present invention. Longer-acting thyroid-stimulating hormone (TSH) compositions that reduce frequency of injection, provide flexible administration regimes, improve therapeutic index and have better efficacy for stimulating thyroid tissue are described herein. In addition, other potency parameters are positively affected by the longer acting rhTSH compositions of the invention including prolonged half life of rhTSH and increased duration of T4 release.

As shown herein, conjugating a polyalkylene glycol polymer, e.g., polyethylene glycol to rhTSH, beneficially altered the pharmacokinetic profile and pharmacodynamic profile of rhTSH. As described in greater detail below, N-terminal PEGylation, lysine PEGylation and carbohydrate polymer attachment of rhTSH were studied. The expectation was that N-terminal PEGylation of rhTSH would result in a TSH with prolonged duration of action, whereas lysine PEGylation and carbohydrate PEGylation of TSH would result in a severe decrease in the potency of rhTSH. The study results described herein show that carbohydrate PEGylation of rhTSH has a positive effect on the potency of rhTSH and both N-terminal PEGylation and lysine PEGylation of TSH greatly reduced the potency of rhTSH.

Accordingly, in one aspect, the invention is directed to a composition comprising recombinant human Thyroid Stimulating Hormone (rhTSH), wherein at least one polyalkylene glycol polymer is attached to a carbohydrate site of the rhTSH at a) a sialic acid group selected from the group consisting of ASN52 of the rhTSH α subunit, ASN78 of the rhTSH α subunit, and ASN23 of the rhTSH β subunit or a combination thereof; or at b)a galactose located at ASN52 of the rhTSH α subunit, ASN78 of the rhTSH α subunit, ASN23 of the rhTSH β subunit or a combination thereof. Also shown herein is site-specific PEGylation of rhTSH which has been mutated to introduce cysteine residues targeted for PEGylation that results in a positive effect on the potency of rhTSH. Thus, in another aspect, the invention is directed to a composition comprising a mutated recombinant human Thyroid Stimulating Hormone (rhTSH) and at least one polyalkylene glycol polymer, wherein the mutated rhTSH comprises a rhTSH in which one or more amino acid residues has been substituted with a cysteine residue, wherein the amino acid residue that has been substituted with cysteine is located on the alpha subunit of rhTSH at an amino acid position selected from the group consisting of ASN66, THR69, and GLY22, and combinations thereof; or is located on the beta subunit of rhTSH at the amino acid position of VAL118; or any combination thereof; and wherein the polyalkylene glycol polymer is attached to the mutated rhTSH at the cysteine residue, and the mutated rhTSH is biologically active. The PEGylated rhTSH compositions provided herein have one or more of the following improved therapeutic index effects relative to rhTSH that is not conjugated to a polyethylene glycol polymer: enhanced solubility, decreased proteolysis, decreased immunogenicity, reduced rate of kidney clearance, prolonged blood circulation lifetime, increased duration of action and altered distribution and absorption.

TSH is a glycoprotein having two subunits, the *alpha* and the *beta* subunit. The α (alpha) subunit *(i.e.,* chorionic gonadotropin alpha) is identical to that of human chorionic gonadotropin, luteinizing hormone, and follicle-stimulating hormone (FSH). The β *(beta)* subunit is unique to TSH, and determines its function.

rhTSH refers to recombinantly synthesized TSH. The recombinant DNA methods described herein are generally those set forth in Sambrook et al., Molecular Cloning: A Laboratory Manual (Cold String Harbor Laboratory Press, 1989, and /or Current Protocols in Molecular Biology (Ausubel et al., eds., Green Publishers Inc., and Wiley and Sons 1994, with Supplements). The term "recombinant" refers to a polynucleotide synthesized or otherwise manipulated *in vitro (e.g.,* "recombinant polynucleotide"), and to methods of using recombinant polynucleotides to produce gene products in cells or other biological systems, and to a polypeptide ("recombinant protein") encoded by a recombinant polynucleotide.

rhTSH used in the methods and compositions described herein can be purified from naturally-occurring mammalian sources, such as bovine, porcine, primate, or human, or alternatively isolated in a recombinant form from non-naturally-occurring sources using methods known in the art, such as described in U.S. Patent Nos. 5,840,566 and 6,365,127.

It is known that conjugation of macromolecules to polymers, (e.g., polysaccharides, polymers of sialic acid, hydroxyethyl starch and polyalkylene glycols (e.g., polypropylene glycol, polybutylene glycol, polyethylene glycol) and other like moieties) can be used for effectively altering the *in vivo* efficacy of drugs by changing the balance between their pharmacodynamic and pharmacokinetic properties. However, such conjugation often leads to loss of binding affinity of the drug and, in effect, loss of potency. In limited cases, a decrease of potency is offset by a longer circulating half-life of the polymer-modified drugs making the resultant pharmacokinetic (PK) and pharmacodynamic (PD) profiles useful for therapy.

While conjugation of polyethylene glycol (PEG), referred to as PEGylation, to certain macromolecules retains most activity, PEGylated hormones and cytokines, whose activity generally require high affinity interactions with cell surface receptors, show significant reduction in activity. Frequently, PEGylation to hormones adversely affects the PD profile and PK profile of the hormone.

Polyethylene glycol (PEG) is a non-antigenic inert polymer that has been shown to prolong the length of time a protein circulates in the body. Typically, these common PEG reagents attach to primary and secondary amines on proteins, generally at lysine residues and/or at the N-terminal amino acid. PEG is commercially available in different sizes and, once attached, can be tailored for individual indications by using the variation of sizes and multiple attachments to a single drug molecule. PEG has been shown to improve plasma half-life of the injected PEGylated protein but as stated above, potency can be lost due to steric hindrance by PEG (Fishburn, C.S., J Pharm Sci 97:4167 (2008)). To avoid potency problems and generate desirable PD and PK profiles, a detailed understanding of structure-function relationship of the target protein to be PEGylated is helpful for generating PEGylated products that retain maximum functional activity.

Recently, progress was made in the determination of the structures of members of the growth hormone superfamily, which has led to potential therapeutics. Understanding how these proteins interact with their targets coupled with the structural information has generated effective drugs by using structural information to design polymer conjugates for maximizing the therapeutic benefits of the protein on their respective target. Unfortunately, structural information for the complex structure of TSH with its receptor is not available; however, the crystal structure of a related protein follicle-stimulating hormone (FSH) complexed to the FSH receptor (FSHR) has been resolved (Fan and Hendrickson, Nature 433:269 (2004)). TSH has high homology with FSH, sharing the identical alpha subunit and similar beta subunit. Likewise, the TSH receptor and FSH receptor are highly homologous. Thus, the FSH-FSH receptor complex may serve as an initial model for TSH interaction with its receptor.

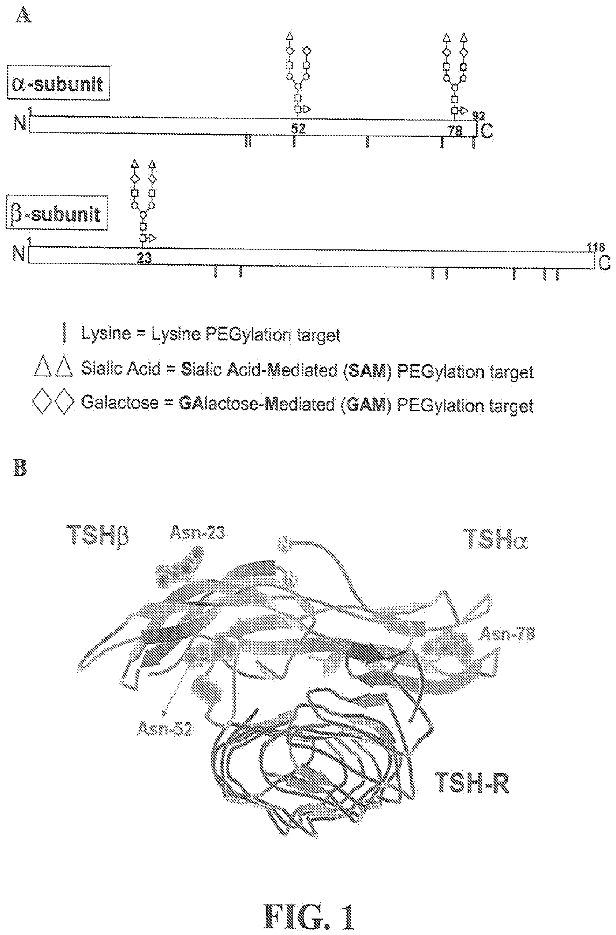

As shown herein, when the TSH sequence was superimposed onto a 3D structural model of the FSH-FSHR complex (PDB coordinate 1XWD), the N-termini appear to be most distant from the receptor interaction region, and thus are likely ideal sites for PEG attachment to minimize loss of receptor binding upon PEGylation (See FIG.1 B). Since there are many lysines in TSH, and some of them are near the receptor binding site, PEG attachment at lysines would be expected to interfere with receptor binding.

TSH is a glycosylated protein. The carbohydrate chains constitute 15-25% of its weight and include three asparagine-linked carbohydrate chains. Two of these chains are found on the alpha subunit of TSH, linked to asparagine 52 (ASN 52) and asparagine 78 (ASN 78), respectively, and the third is on the beta subunit of TSH, linked to asparagine 23(ASN 23). Asparagine-linked carbohydrate chains are potential PEG conjugation sites, however, on the TSH protein their respective proximities to the receptor interaction region, in particular, ASN 52, suggests that PEG conjugation at such sites would likely have a negative effect on receptor binding. Thus, selecting potential sites for conjugation of polymers to TSH presented much uncertainty.

Furthermore, deglycosylation of TSH has long been known to result in loss of efficient signal transduction upon receptor binding (Szkudlinski et al., Physiol Rev. 82:473 (2002)). While removal of the terminal sialic acids from the carbohydrates of TSH improved *in vitro* activity, greatly reduced circulation time *in vivo was* observed, presumably due to clearance by hepatic asialoglycoprotein receptor. Thotakura, N.R., Szkudlinski, M.W. and Weintraub, B.D., Giycobioiogy 4, 525-533 (1994). Deleting individual carbohydrate sites also enhanced *in vitro* functional activity, especially for the alpha chain amino acid position 52, again suggesting that the carbohydrates may be close enough to the receptor for electrostatic repulsion between the negative charges of the sialic acids and negative charges on the receptor. All together, this structure function analysis of TSH indicated that N-terminal PEGylation is far more preferable than carbohydrate PEGylation to minimize effect on functional potency. As described herein, N-terminal PEGylation, lysine PEGylation and carbohydrate polymer attachment were studied.

Accordingly, carbohydrate polymer methodology to produce polymer conjugated rhTSH is one aspect of the present invention. In one embodiment, the polymer is polyalkylene glycol. As used herein, "polyalkeylene glycol (PAG)" includes polyethylene glycol (PEG), polypropylene glycol (PPG), polybutylene glycol, and the like. The polymers can be linear or branched. The PAG is attached covalently to a molecule. As used in the present context, the term "attachment" or "attached" refers to the coupling or conjugation of a site or moiety of the TSH protein and a polymer, such as a polyalkylene glycol, e.g., either directly covalently joined to one another, or else is indirectly covalently joined to one another through an intervening moiety or moieties, such as a bridge, spacer, or linkage moiety or moieties. An attached or conjugated polymer to TSH is differentiated from the polymer being admixed, commingled, or in solution with the TSH.

"Carbohydrate site" refers to a carbohydrate side chain found on rhTSH. The site can be a naturally glycosylated site or a site that has been enzymatically provided. In certain embodiments, the carbohydrate site is specifically selected to meet desired criteria for optimal rhTSH interaction with the receptor or for other functional requirements such as folding or mobility of the protein. The carbohydrate site is available for attachment of a polymer moiety such as PEG. Typical carbohydrate sites on the protein are asparagine, serine or threonine. rhTSH has three asparagine-linked carbohydrate chains.

In certain embodiments, a single polymer is attached to rhTSH. In other embodiments, multiple polymers are attached the rhTSH. The multiple attached polymers can be of a single specie or multiple species. For example, in the case where a single PEG molecule is attached to the protein the protein is referred to as "monoPEGylated" and in the case where more than one PEG molecule is attached the protein is referred to as "multiPEGylated". When the protein is multiPEGylated, an individual PEG attached to the protein (vis a vis other attached PEGs) can have the same or a different molecular weight, and can be of a linear or branched structure. The molecular weight range of the PEG molecule is 3,000-100,000 Daltons. In certain embodiments, the molecular weight of the PEG attached is 5kD, 10kD, 20kD, 30kD, 40kD, or 60kD or combinations thereof.

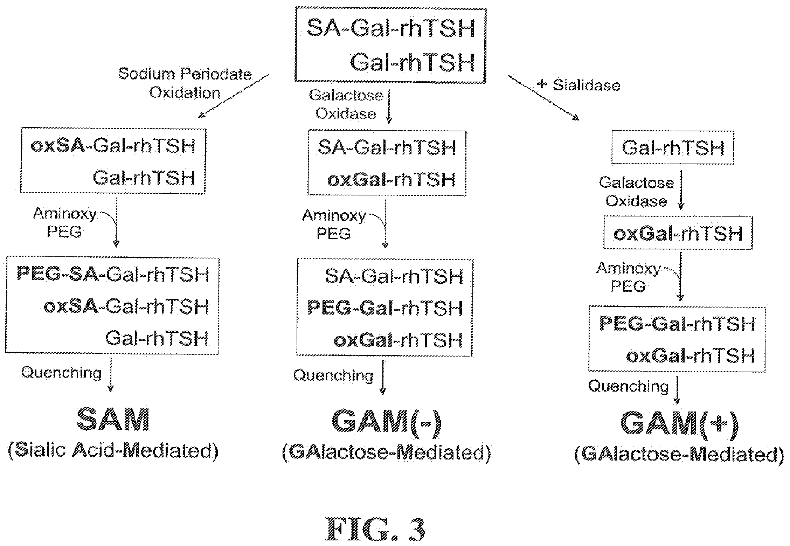

Provided herein are three alternative schemes for carbohydrate PEGylation of TSH: (i) sialic acid-mediated PEGylation (referred to as "SAM"), (ii) galactose-mediated PEGylation (referred to as "GAM(-)"), and (iii) sialic acid removal coupled with galactose-mediated PEGylation (referred to as "GAM(+)"). Briefly, as shown in FIG. 3, in sialic acid mediated SAM PEGylation, the sialic acid is oxidized, followed by chemical attachment of PEG. In GAM(-) PEGylation, the galactose moiety is oxidized, followed by chemical attachment of PEG. And in GAM(+), a sialic acid moiety is enzymatically removed, and the exposed galactose residue is then oxidized followed by the chemical attachment of PEG.

Site-specific methods of PEGylation to rhTSH are also included in the present invention. One such method attaches PEG to cysteine residues using cysteine-reactive PEGs. A number of highly specific, cysteine-reactive PEGs with different reactive groups (e.g., maleimide, vinylsulfone) and different size PEGs (2-40 kDa) are commercially available. At neutral pH, these PEG reagents selectively attach to "free" cysteine residues, *i.e.,* cysteine residues not involved in disulfide bonds in the target protein. Alternatively, one of two cysteines involved in a native disulfide bond may be deleted or substituted with another amino acid, leaving a native cysteine (the cysteine residue in the protein that normally would form a disulfide bond with the deleted or substituted cysteine residue) free and available for chemical modification. Preferably the amino acid substituted for the cysteine would be a neutral amino acid such as serine or alanine.

Through *in vitro* mutagenesis using recombinant DNA techniques, additional cysteine residues can be introduced at any useful position on the protein. The newly added "free" cysteines can then serve as sites for the specific attachment of a PEG molecule using cysteine-reactive PEGs. The added cysteine residue is a substitution for an existing amino acid in a protein. Cysteine residues can be added preceding the amino-terminus of the protein, after the carboxy- terminus of the protein, or inserted between two amino acids in the protein. The term "mutant TSH," as used herein refers to a TSH where one or more amino acids of the wild-type TSH have been substituted with another amino acid that permits the formation of one or more glycosylation sites on the TSH molecule. Accordingly the amino acid substitution of TSH enables site-specific coupling of at least one polymer, such as a polyalkylene glycol. For example, site-specific coupling with PEG molecules to the mutant rhTSH allows the generation of a rhTSH that possesses the pharmacodynamic and pharmacokinetic benefits of a polyethylene-glycosylated rhTSH.

In particular embodiments, amino acid substitution with cysteine can be at the positions shown for the mutants in Tables 1 and 2.

In a preferred embodiment, the substitution does not substantially change the structural characteristic of native TSH. The amino acid residue that has been substituted with cysteine is located at an amino acid position on the alpha subunit of recombinant human TSH selected from the group consisting of ASN66, THR69, and GLY22, and combinations thereof; or is located on the beta subunit of rhTSH at the amino acid position of VAL118; or any combination thereof; and the polyalkylene glycol polymer is attached to the mutated rhTSH at the cysteine residue, and the mutated rhTSH is biologically active is described.

The rhTSH compositions described herein are biologically active. "Biologically active" means that the rhTSH compositions of the invention have one or more of the following effects: longer duration of action, prolonged half-life, increased duration of T4 release, higher AUEC, positive shift in Tmax, and a lower peak-to-trough exposure that can reduce side effects. In certain embodiments, the compositions are compared to a control and the effect is substantially similar, somewhat less or somewhat greater or substantially greater. The biological activity of rhTSH compositions produced according to the present invention can be assessed using a variety of techniques known to those of skill in the art.

When assessing the biological activity of a rhTSH composition of the present invention, the biological activity can be compared to a control. As appreciated by one of skill in the art, a variety of suitable controls can be used. In one embodiment "control" as used herein refers to native (wild-type) TSH or rhTSH.

The compositions of this invention, when administered to a patient in need thereof (e.g., a thyroid cancer patient who had near-total or total thyroidectomy), will provide a blood serum concentration of rhTSH that has been tailored for the indicated use. In a certain embodiment, the duration of action for the compositions described herein is increased by at least 2-fold over rhTSH. In another embodiment, the duration of action for the TSH compositions is increased by at least 3-fold over rhTSH. Such embodiments having increased duration of action effectively reduce the frequency of administration while maintaining comparable efficacy to the current formulation of rhTSH.

In one embodiment, the compositions provide longer half-life (t1/2), compared to rhTSH. In particular embodiments, the t1/2 is up to 23-fold longer than rhTSH in rat.

In another embodiment, the T4 level is shown to have a greater sustained effect over rhTSH. In rat, T4 level in rhTSH group was back to vehicle level by 48 hours post-dose whereas in one embodiment T4 was sustained for 168 hours post-dose.

An "effective Tmax" as used herein refers to a "Time of the Peak Height Concentration", which is characteristic of the composition in reference. An "effective Cmax" as used herein refers to a "Peak Height Concentration", which is characteristic of the composition in reference. In many situations, an effective Tmax and Cmax provide a blood (or serum or plasma) concentration time curve in which the concentration of a drug is in a therapeutic range. "t1/2" refers to the duration of action of a drug is known and the period of time required for the concentration or amount of drug in the body to be reduced by one-half.

The nucleic acid sequence of the alpha subunit of human TSH is:

gctcctgatgtgcaggattgcccagaatgcacgctacaggaaaacccattcttctcccagccgg
gtgccccaatacttcagtgcatgggctgctgcttctctagagcatatcccactccactaaggtcca
agaagacgatgttggtccaaaagaacgtcacctcagagtccacttgctgtgtagctaaatcatat

aacagggtcacagtaatgggggtttcaaagtggagaaccacacggcgtgccactgcagtac
ttgttattatcacaaatct (SEQ. ID NO: 1).

The amino acid sequence of the alpha subunit of human TSH is: 
APDVQDCPECTLQENPFFSQPGAPILQCMGCCFSRAYPTPLRSKKT
MLVQKNVTSESTCCVAKSYNRVTVMGGFKVENHTACHCSTCYYHK
S
 (SEQ ID NO: 2)

The nucleic acid sequence of the beta subunit of human TSH is:

ttttgtattccaactgagtatacaatgcacatcgaaaggagagagtgtgcttattgcctaaccatca
acaccaccatctgtgctggatattgtatgacacgggatatcaatggcaaactgtttcttcccaaat
atgctctgtcccaggatgtttgcacatatagagacttcatctacaggactgtagaaataccaggat
gcccactccatgttgctccctatttttcctatcctgttgctttaagctgtaagtgtggcaagtgcaatac
tgactatagtgactgcatacatgaagccatcaagacaaactactgtaccaaacctcagaagtct
tatctggtaggattttctgtc (SEQ ID NO: 3)

The amino acid sequence of the beta subunit of human TSH is: FCIPTEYTMHIERRECAYCLTINTTICAGYCMTRDINGKLFLPKYALS QDVCTYRDFIYRTVEIPGCPLHVAPYFSYPVALSCKCGKCNTDYSD CIHEAIKTNYCTKPQKSYLVGFSV (SEQ ID NO: 4)

As used herein, the term "amino acid residues corresponding to amino acid residues of the subunits of TSH is intended to indicate the amino acid residues corresponding to the sequence of wild-type TSH subunits (SEQ ID NOs: 2 and 4 ) when the sequences are aligned. Amino acid sequence homology/identity is conveniently determined from aligned sequences, using a suitable computer program for sequence alignment, such as, *e.g.,* the ClustalW program, version 1.8, 1999 (Thompson et al., 1994, Nucleic Acid Research, 22: 4673-4680).

### EXEMPLIFICATION

### Example 1. Sialic Acid-Mediated (SAM) PEGylation of rhTSH

Sodium periodate oxidation: 25 mM sodium periodate (Sigma, 311448) in 100 mM sodium acetate, pH 5.6 was added to 4.5 mg/ml TSH in 100 mM sodium acetate, pH 5.6 to final concentrations ranging from 0.2 mM to 2mM, in a glass vial wrapped in aluminum foil. The mixture was gently shaken on ice in the dark for 30 minutes. After 30 minutes, 50% glycerol was added to 3% of the reaction volume and then shaken for 15 minutes. The mixture was then buffer exchanged to 100 mM sodium acetate, pH 5.6, and concentrated to a TSH concentration of at least 4.3 mg/ml in order to perform the PEGylation.

PEGylation: The appropriate size aminoxy PEG (100 mg/ml in dH₂O) was added to the oxidized TSH to varying (PEG:Protein) molar ratios. The reaction volume was adjusted with 100 mM sodium acetate, pH 5.6, to a final TSH concentration of 4 mg/ml. The mixture was then incubated at 25°C for 16 hours or at 8°C for 16 hours with gentle shaking. After incubation, a 50 molar excess of 0.05M hydroxylamine was added to quench the reaction mix and incubated at 25°C for 6 hours with gentle shaking.

### Example 2. Galactose-Mediated (GAM(+)) PEGylation of Desialylated rhTSH

Neuraminidase Treatment: 20 mU neuraminidase (His6-Clostridium neuramindase (520 mU/ul)) was added per mg TSH and incubated at 37°C for 6 hours.

Catalase/Galactose Oxidase Treatment: 2 U Catalase (Sigma 442 U/µl) per mg TSH was added to the neuraminidase treated TSH. 4 µg galactose oxidase (Worthington GAO, 1.2 mg/ml) per mg TSH was added to the mixture and then incubated at 37°C for 16 hours. After incubation the mixture was buffer-exchanged and concentrated into 100 mM sodium acetate, pH 5.6, to a concentration of at least 5.5 mg/ml in order to perform the PEGylation.

PEGylation: The appropriate size aminoxy PEG (100 mg/ml in dH₂O) was added to the oxidized and buffer-exchanged TSH to the (PEG:Protein) molar ratio of 1:1. The reaction volume was adjusted with 100 mM sodium acetate, pH 5.6, so that the final TSH concentration in the reaction was 5 mg/ml. The mixture was then incubated at 25°C for 16 hours with gentle shaking. A 50 molar excess of 0.05M hydroxylamine (m.w. 69.49) was then added to the reaction mix and incubated at 25°C for 6 hours with gentle shaking.

### Example 3. Galactose-Mediated (GAM(-)) PEGylation of rhTSH

Catalase/Galactose Oxidase Treatment: 2 U Catalase (Sigma 442 U/µl) per mg and 4 µg galactose oxidase (Worthington GAO, 1.2 mg/ml) per mg were added to TSH. The mixture was then incubated at 37°C for 16 hours. After incubation the mixture was buffer-exchanged and concentrated into 100 mM sodium acetate, pH 5.6, to a final concentration of at least 5.5 mg/ml in order to perform the PEGylation.

PEGylation: The appropriate size aminoxy PEG (100 mg/ml in dH₂O) was added to the oxidized and buffer-exchanged TSH to the (PEG:Protein) molar ratio of 1:2. The reaction volume was adjusted with 100 mM sodium acetate, pH 5.6, so that the final TSH concentration in the reaction was 5 mg/ml. The mixture was then incubated at 25°C for 16 hours with gentle shaking. A 50 molar excess of 0.05M hydroxylamine (m.w. 69.49) was then added to the reaction mix and incubated at 25°C for 6 hours with gentle shaking.

### Example 4. N-terminal PEGylation of rhTSH

Appropriate size aldehyde PEG (100 mg/ml in reaction buffer) was added to a final concentration of 5 mg/ml rhTSH at varying (PEG:Protein) ratios in 100mM sodium acetate, pH 5 or pH 5.6, with 20mM sodium cyanoborohydride. Incubation was done at 25°C for 16 hours or 8°C for up to 2 days, before quenching the reaction with 0.1 volume of 1M Tris, pH 7.5, for 3 hours at 25°C.

PEGylation at the N-terminus yielded conjugates that lost greater TSH receptor binding affinity than carbohydrate conjugation. N-terminal mono-PEGylation with 40kD PEG resulted in an 11.1 -fold decrease of *in vitro* TSH receptor binding affinity, compared to the rhTSH control.

### Example 5. Lysine PEGylation of rhTSH

Appropriate size NHS (N-hydroxysuccinimide) PEG (50mg/ml in dH₂O)was added to a final concentration of 0.8 mg/ml rhTSH at varying (PEG:Protein) ratios (e.g., (0.5:1), (1;1), (2:1), (4:1), (8:1)) in PBS (phosphate-buffered saline) buffer, pH 7.2. Incubation was done at 25°C for 1.5 hours.

Lysine conjugation with N-hydroxysuccinimide ester (NHS) PEG was explored. For 40 kDa PEGs, different (PEG:protein) ratios and different incubation times were tested in the PBS (phosphate-buffered saline) buffer, pH 7.2. Final TSH concentration was 0.8 mg per ml. PEGylation was done at 25°C or 37°C for 1.5 hours or 19.5 hours. The short incubation time tested (1.5 hour) showed the same results as long incubation time (19.5 hours). This was presumably due to a rapid hydrolysis of NHS PEG in aqueous solution. The extent of PEGylation depended on the (PEG:Protein) molar ratios, with higher PEG molar excess producing more multi-PEGylated conjugates.

Size exclusion chromatography (SEC) fractions of mono-PEGylated species were collected and submitted for *in vitro* TSH receptor binding assay. *In vitro* TSH receptor binding assay results showed that mono-40kDa NHS PEG-TSH (Lysine PEGylation) has 31.2-fold lower affinity than the control TSH, starting material, while mono-40kDa aminoxy PEG-TSH (SAM PEGylation) has 5.3-fold lower affinity.

### Example 6. Production of Cysteine Mutants for Site-specific conjugation

TSH single mutants were designed and prepared to introduce cysteines for site-specific PEGylation. These mutants were designed to minimize its effect on protein folding, receptor binding and for their potentials to be effectively conjugated.

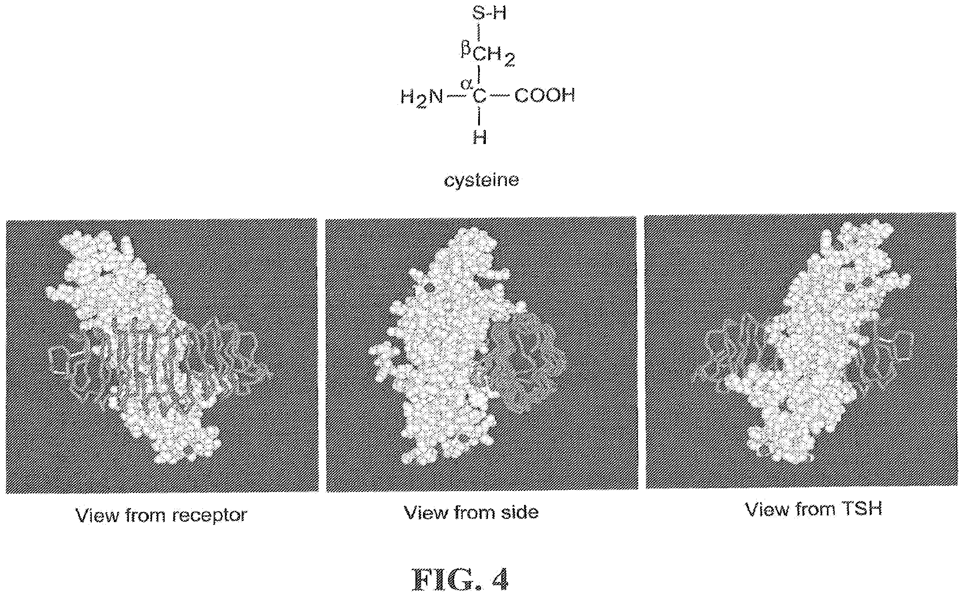

The following considerations were applied to design sites to introduce cysteine mutants based on a structural model of TSH/TSH receptor: 1) The mutation site should not be located at or adjacent to a receptor binding site; 2) The mutation site should not be located at or adjacent to an alpha/beta subunit dimerization interface; 3) The mutation site should not be located at or adjacent to a disulfide bond; 4) Avoid sites that when mutated, result in dramatic loss in specific activity based on reported literature; 5) The mutation site should be solvent exposed for subsequent PEGylation; 6) Select sites that would evenly cover most of the TSH surface opposite of the receptor binding site to fully evaluate PEGylation feasibility at each region. Some of these considerations are summarized in Table 1. The beta-carbon positions of the selected mutants were exposed to solvent in our structure model as shown in FIG. 4. This predicts that these positions are likely to be accessible for PEGylation reagents when mutated to cysteine. The first three sites selected in Table 1 were native glycosylation sites in TSH.

**Table 1**

| **Mutant ID** | **Mutant** | **Subunit** | **Previously Reported Mutagenesis Data** |
|---|---|---|---|
| 1 | N52C(a) | alpha | 6 x ↑ in specific activity when changed to Gin |
| 2 | N78C(a) | alpha | 2-3 x ↑ in specific activity when changed to Gln |
| 3 | N23C(b) | beta | 2-3 x ↑ in specific activity when changed to Gln |
| 4 | V118C(b) | beta | |
| 5 | M71C(a) | alpha | < 2 x ↓ in specific activity when oxidized |
| 6 | N66C(a) | alpha | slight increase in specific activity when changed to Lys |
| 7 | T69C(a) | alpha | |
| 8 | G22C(a) | alpha | |
| 9 | T21C(b) | beta | |
| 10 | E63C(b) | beta | |
| 11 | D56C(b) | beta | Slight change in specific activity with when mutated |

DNAs encoding rhTSH genes (including its signal peptides) were synthesized and cloned into a Gateway entry vector (for example, pDONOR221). Oligonucleotide-based site-directed mutagenesis was used to introduce Cys mutations at multiple sites on both TSH subunits. The resulting wild-type and mutant vectors were shuffled into expression vectors (for example, pCEP4.DEST) via Gateway cloning. Proteins were prepared from transiently transfected HEK293 cell media and characterized by biochemical and cell-based assays, e.g., gel electrophoresis, Western blotting, SEC-HPLC chromatography, PEG modification yield and cell reporter assays.

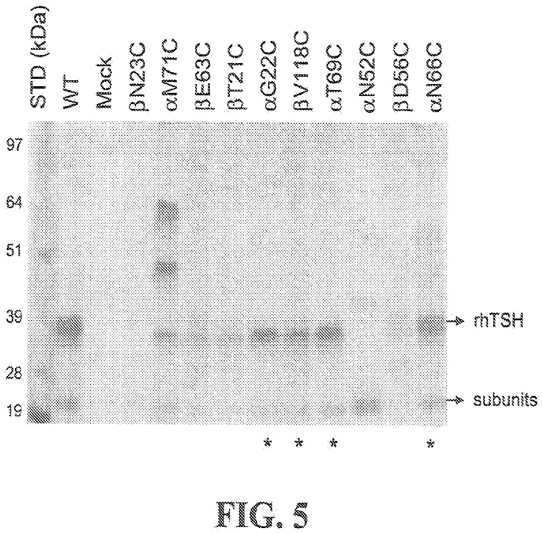

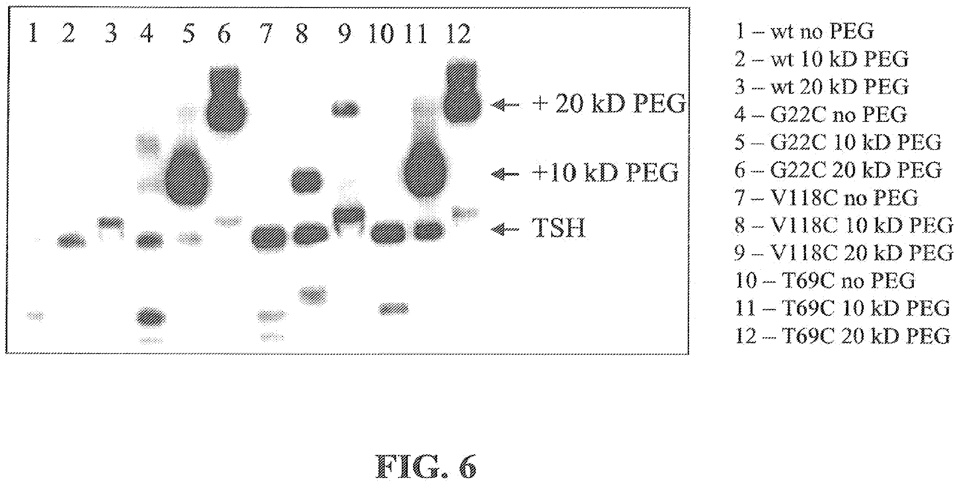

The results were evaluated and compared for their expression level, aggregation (dimerization) tendency, PEGylation efficacy and perturbation to TSH function. For example, silver stain analysis of purified mutant TSH revealed that mutants TSH N66C(alpha), TSH G22C(alpha), TSH M71C(alpha), TSH T69C(alpha), and TSH V118C (beta) had the best expression levels (See FIG. 5). PEGylation experiments suggested that TSH G22C(alpha), TSH N66C(alpha), TSH T69C(alpha), and TSH V118C (beta) were effectively PEGylated (See FIG. 6 for representative PEGylation results). M71C(alpha) seemed to form aggregates in the PEGylation incubation. (Data not shown). The decision was made to move selected mutants (e.g., G22C) forward for large scale production and in vivo studies (See Table 2).

**Table 2**

| # | Mutant | Subunit | Expression | Monomer | PEGylation | Away from TSHR | Selected leads |
|---|---|---|---|---|---|---|---|
| 1 | WT | | *** | *** | | | |
| 2 | N52C(a) | alpha | ND | | | | |
| 3 | N78C(a) | alpha | ND | | | | |
| 4 | N23C(b) | beta | ND | | | | |
| 5 | V118C(b) | beta | * | *** | * | | |
| 6 | M71C(a) | alpha | ** | * | | | |
| 7 | N66C(a) | alpha | ** | ** | ** | ** | |
| 8 | T69C(a) | alpha | ** | ** | *** | ** | #2 |
| 9 | G22C(a) | alpha | ** | *** | *** | *** | #1 |
| 10 | T21C(b) | beta | ND | | | | |
| 11 | E63C(b) | beta | ND | | | | |
| 12 | D56C(b) | beta | ND | | | | |

Larger scale Cys mutants were prepared from CHO pools. DNAs encoding the wild-type (WT) and mutant rhTSH genes were codon-optimized and synthesized. These genes were cloned into CHO expression vectors (for example, pGEN600, pGEN620) for transient transfection into CHO cells. Transfected CHO cells were amplified with methotrexate (MTX) selection. The resulting CHO pools were used for scale-up protein production.

The cysteine TSH mutants were found to be capped at the introduced cysteine after expression and purification, thus additional reducing methodology was needed to create PEGylated mutant TSH. The mutant TSH first needed to be reduced with a mild reductant to release the cap from the introduced cysteine without irreversibly breaking the native disulfide bonds that would inactivate the protein.

Accordingly, multiple methods were employed in an effort to reduce the capped cysteine. For example, various concentrations of TCEP (Tris(2-carboxyethyl)phosphine HCI) solution were incubated with rhTSH mutants for reduction. While removal of the cysteine cap was effective using this method, there also appeared to be some reduction of other disulfide bonds which led to non-specific PEGylation later. Another reducing method using immobilized beads with TCEP seemed to be milder in reducing rhTSH however, this method also resulted in some low levels of disulfide reduction. We found that 1-10 mM cysteine (as a reductant) was particularly effective to reduce the capped Cys without breaking the existing disulfide bonds in rhTSH.

The cysteine reductant was removed together with the cap to allow the disulfides to reform. The "de-capped" introduced cysteine was then selectively conjugated to a cysteine-reactive PEG reagent. Although this method has been demonstrated in other proteins such as FVIII (Mei et al., Blood 116:270-9 (2010)) and antibody fragments (Yang et al., Protein Engineering 16:761-770 (2003)), however, there are no known reports of attempting this on a cysteine knot-containing protein. Whereas the previous reported examples had only 1-2% cysteine content, TSH has 11% cysteine content (23/210 amino acids), which makes the introduction of a 24^{th} cysteine into TSH without scrambling the native disulfides (and thus dramatically lowering receptor binding affinity) particularly difficult. Consequently, we had to screen multiple positions for introducing the cysteine mutation, with only a few that could be successfully conjugated with a PEG.

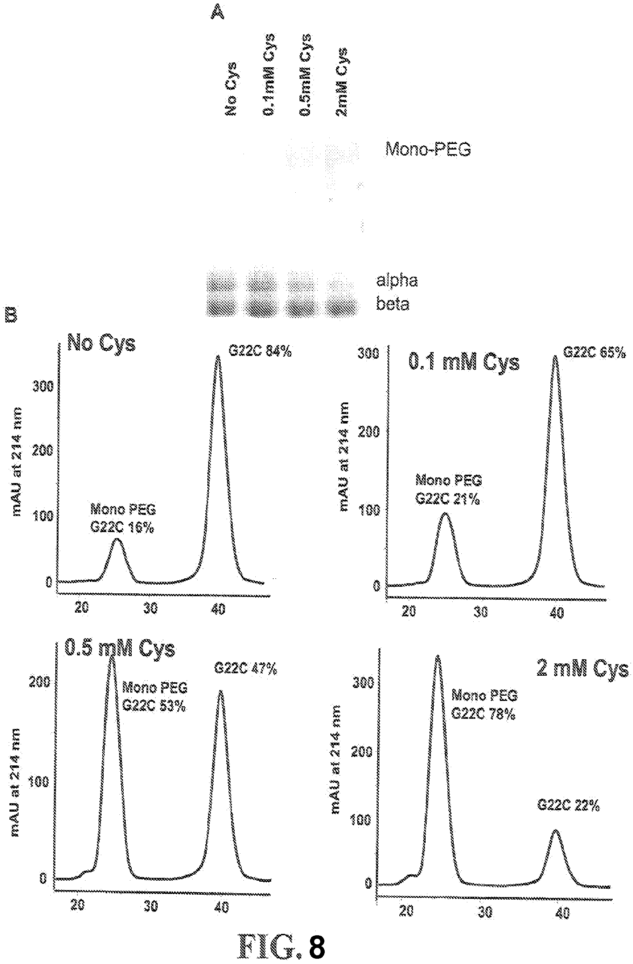

**Site-specific Cys PEGylation:** G22C rhTSH was produced from CHO cells for site-specific PEGylation. Cysteine was added to G22C rhTSH (1-2mg/ml) to a final concentration of 2mM after optimization experiments shown in FIG. 8. The protein was incubated overnight at 4ºC to remove the cap on Cys22. The mixture was dialfiltered into PEGylation buffer (10 mM sodium phosphate, 2mM EDTA, pH 7.0). PEG was added to the protein to get 5x molar excess and incubated at 25ºC for 2 hours. The PEGylation was stopped with 2x cysteine and the yield was checked by SEC-HPLC. The pH of the action mixture was lowered to pH5.0 and then loaded onto a monoS column for purification.

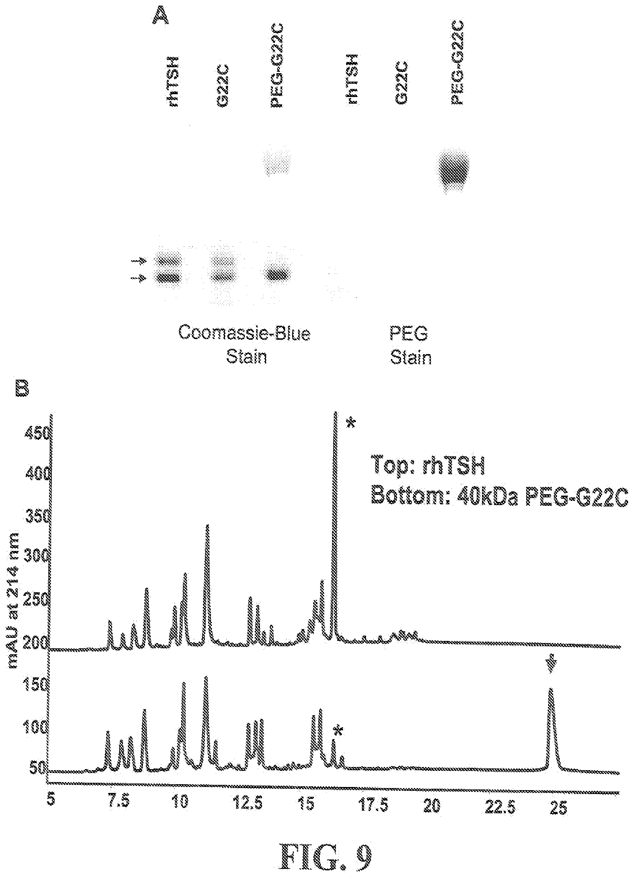

The SEC-HPLC profiles of several PEGylation reactions are shown in FIG.8. Approximately 75% mono-PEGylated G22C TSH was obtained, which is very effective conjugation. Confirmation of subunit- and site-specific conjugation of G22C is shown in FIG. 9A - FIG. 9B.

### Example 7. MonoS AKTA Purification of PEGylated rhTSH

Samples were purified over a monoS column (GE Healthcare) and eluted using a gradient with 10mM sodium acetate pH5 and 10mM sodium acetate, 1 M sodium chloride pH5 at a flow rate of 4ml/min. The gradient started with 0% mobile phase B (10mM sodium acetate, 1 M sodium chloride pH5) then increased to 50% mobile phase B over 25 column volumes followed by 100% mobile phase B to wash the column.

### Example 8. SDS-PAGE Analysis and Staining

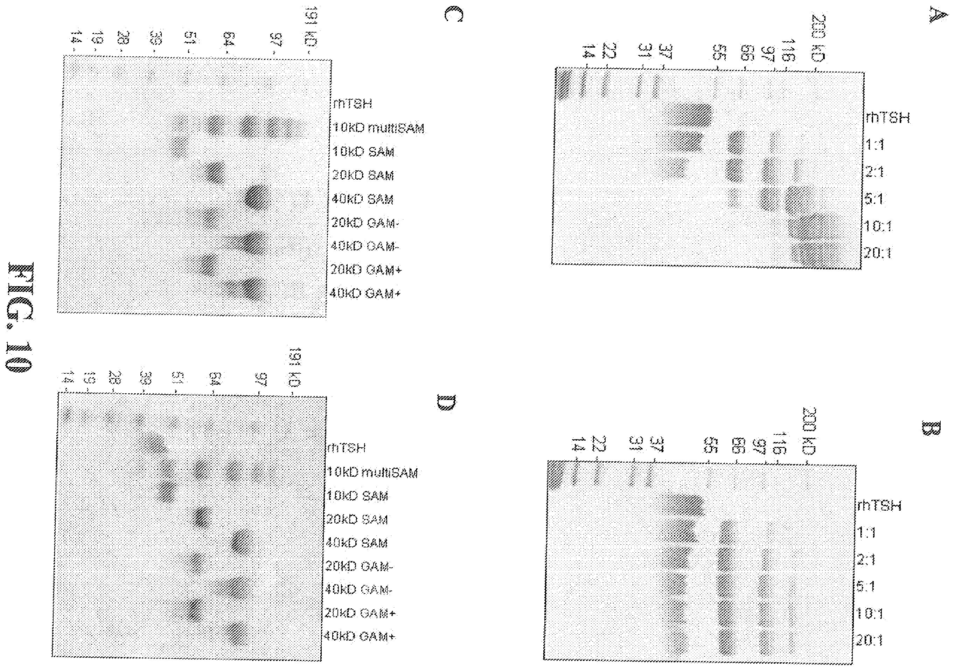

Pre-poured gradient gels (4-12% Bis Tris, Invitrogen) were loaded with 4-5µg TSH. MOPS (3-(N-morpholino)propanesulfonic acid ) running buffer (Invitrogen) was prepared. The electrophoresis apparatus was placed in an ice bucket with ice. The gel ran for approximately 50 minutes at 200V and was then rinsed three times, 5 minutes each with distilled water. 50 ml 5% barium chloride was added to the gel and then shaken for 10 minutes. The barium chloride was removed by rinsing the gel for 5 minutes with distilled water. The distilled water was then removed and the gel was first stained for PEG with 1x potassium iodide/ iodine solution until the bands were visible. The gel was then destained with distilled water and scanned immediately. For protein staining, Coomassie destain (10% acetic acid, 20% methanol) was used to remove all traces of the PEG stain and the gel was then stained with Coomassie blue stain. SDS-PAGE analysis results of purified carbohydrate PEGylated rhTSH conjugates are shown in FIG. 10C (PEG staining) and FIG. 10D (Coomassie staining).

### Example 9. SEC-HPLC Analysis of PEGylated rhTSH

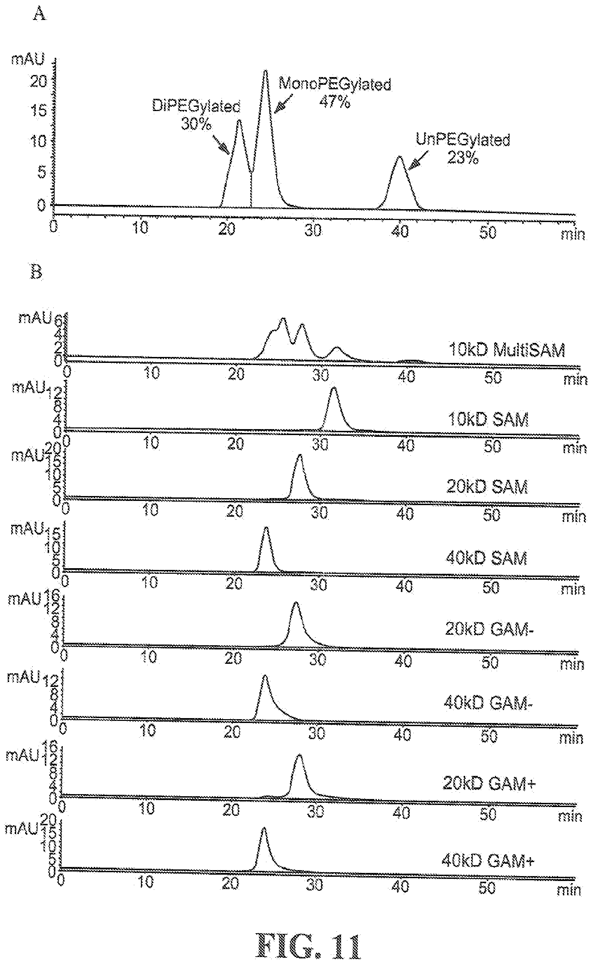

Samples were run on a Superdex 200 10/300GL column (GE Healthcare) and eluted at a flow rate of 0.4ml per min. in 50mM sodium phosphate, 150mM sodium chloride buffer pH7. Representative SEC-HPLC profiles of the 40 kD SAM reaction and purified PEGylated rhTSH conjugates are shown in FIG. 11A and FIG. 11B, respectively.

### Example 10. Peptide Mapping and N-terminal Sequencing of PEGylated rhTSH

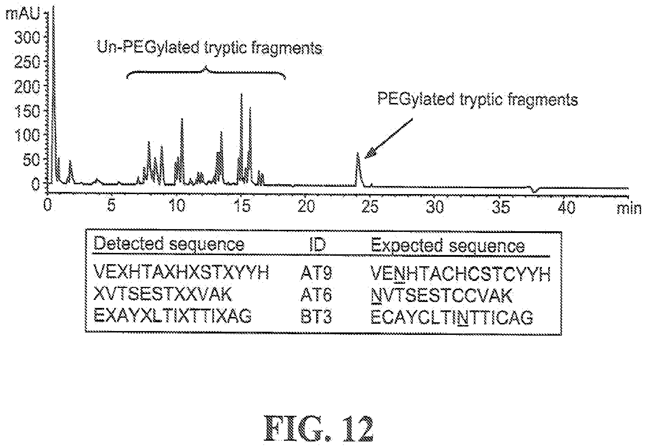

Twenty micrograms of each sample was diluted into 0.1 M Tris, pH 8.5 containing 6 M guanidine hydrochloride and 38 mM dithiothreitol, overlaid with nitrogen, and incubated at 25 °C overnight. After incubation, iodoacetamide was added at 50 mM. The samples were then overlayed with nitrogen and incubated at 25 °C for 2 hours. The alkylation reaction was quenched by adding 1/1 (v/v) 0.25% trifluoroacetic acid and the samples were dialyzed into 0.1 M Tris, pH 8.5 using 3,500 MWCO Slide-A-Lyzer mini dialysis units (Thermo Scientific). Samples were digested with 1:25 (Enzyme:Sample) ratio overnight at 37 °C. Digest reactions were quenched with 1/1 (v/v) 0.25% trifluoroacetic acid. Trypsin-digested samples were fractionated using an Agilent 1200 HPLC equipped with an automated injector and fraction collector, a binary solvent pump, a thermostatted column compartment, and a variable wavelength detector. Samples were loaded onto a Poroshell 300SB-C8 column (2.1 x 75 mm, 5 µm particles, Agilent Technologies, CA) that was held at 50°C and pre-equilibrated in 98% solvent A (0.1% trifluoroacetic acid in water) and 2% solvent B (0.08% trifluoroacetic acid in acetonitrile). Tryptic peptides were eluted using a linear gradient of solvent B from 2% to 60% over 25 min at a flow rate of 0.4 ml/min. PEGylated fragments, eluting at a greater %B than the unPEGylated fragments, were collected and dried in a centrifugal concentrator (Thermo Scientific, MA). Automated N-terminal sequence analysis was performed using a Procise protein sequencer (Applied Biosystems, CA). Two hundred pmol of sample and control were subjected to 18 cycles of the automated Edman degradation using the preprogrammed pulsed liquid method. FIG. 12 shows the peptide map and N-terminal sequencing results of the 40 kD SAM conjugate. Only 3 tryptic glycopeptides (AT9, AT6, BT3) were detected, indicating the site specificity of carbohydrate PEGylation. Underlined N corresponds to N-linked glycosylation site.

### Example 11. Determination of the relative amount of PEGylation on each subunit

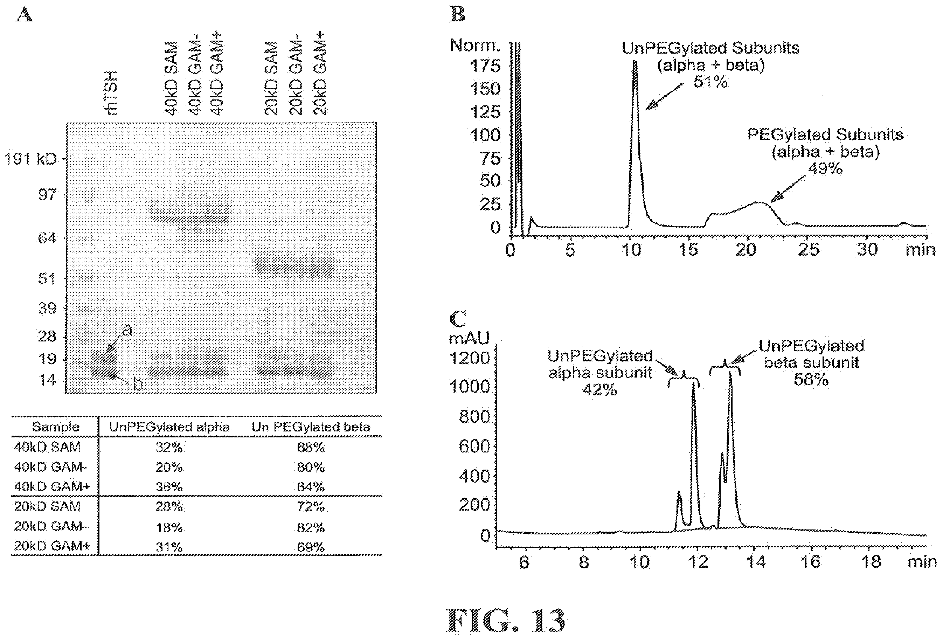

Subunit-specific PEGylation was calculated by measuring the relative amount of unPEGylated α and β subunits after isolating them from the PEGylated subunits, using two consecutive reversed-phase HPLC runs. This method of inference was used because chromatographic conditions that resolve PEGylated α and β subunits could not be identified. Samples (100 µg) were concentrated to 20 µl by centrifugal ultrafiltration and then denatured in 6 M guanidine hydrochloride, 10 mM sodium phosphate, 100 mM sodium chloride, pH 7.0. After overnight incubation at 25°C, the samples were loaded onto a Poroshell 300SB-C8 column (2.1 x 75 mm, 5 µm particles, Agilent Technologies, CA) pre-equilibrated with 75% solvent A (10 mM sodium phosphate, pH 6.5) and 25% solvent B (40% 10 mM sodium phosphate, pH 6.5, 60% acetonitrile). The column was eluted with 25-50%B over 5 min followed by 50-75%B over 20 min at 0.4 ml/min at 25°C. The peak fraction corresponding to unPEGylated TSH subunits (~9.5-12.5 min) was collected (FIG. 13B) in its entirety and concentrated to less than 50 µl by centrifugal ultrafiltration. The samples were adjusted to 50 µl with water and then reduced by addition of 4.7 µl of 2 M dithiothreitol and 150 µl of 6 M guanidine hydrochloride, 0.1 M Tris, pH 8.5, overlayed with nitrogen and incubated at 25°C overnight. Free thiols were then alkylated by adding 9.3 µl of 2 M iodoacetamide, overlaying with nitrogen, and incubating for 2 hr at 25°C. The alkylation reaction was quenched by adding 150 µl of 0.25% trifluoroacetic acid. Reduced and alkylated unPEGylated TSH subunits were profiled by the second reversed-phase HPLC run. The HPLC column setup was identical to that indicated above with the exception that solvent A consisted of 0.1% trifluoroacetic acid in water and solvent B consisted of 0.08% trifluoroacetic acid in acetonitrile and the column was held at 50°C. The column was eluted with a linear gradient of 2-75%B in 15 min at 0.3 ml/min. The relative percentage of unPEGylated α vs. β subunits was determined by integration of the resulting A214 nm chromatograms (FIG. 13C) from triple injections per sample. The relative percentage of PEGylated α vs. β subunits was then taken as the inverse of these values. This analysis showed the fraction of α-subunit modified in the 40kD monoPEGylated GAM-, GAM+ and SAM conjugates to be 77%, 66%, and 58%, respectively, in agreement with the results obtained by SDS-PAGE (FIG. 13A).

### Example 12. In vitro Receptor Binding Assay

Purified PEGylated conjugates were analyzed by *in vitro* porcine TSH receptor binding assay using the TSH Receptor Autoantibody 2nd Generation ELISA kit from RSR Limited (Kronus, Star, ID). Instead of using the biotinylated human monoclonal autoantibody to the TSH receptor provided by the kit, we biotinylated Thyrogen^{®} (rhTSH) to use for competitive inhibition of binding to TSH receptor. Binding of biotinylated rhTSH to immobilized porcine TSH receptor was inhibited by either rhTSH control or PEGylated rhTSH conjugates and IC₅₀ values were measured.

Thyrogen^{®} was biotinylated with 1.7 to 1.8 biotins per protein using the ChromaLink^{™} Biotin Labeling Reagent according to the manufacturer's protocol (QED Bioscience Inc., San Diego, CA) and buffer exchanged into 50mM sodium phosphate, 150mM sodium chloride pH 7.0 with a Zeba^{™} Desalt Spin Column (Thermo Scientific, Rockford, IL). *In vitro* measurement of receptor binding was performed by competition of biotinylated Thyrogen^{®} and PEG-rhTSH conjugate for binding to porcine TSH receptor immobilized onto 96-well plates supplied with the TSH Receptor Autoantibody 2^{nd} Generation ELISA kit from RSR Limited (Kronus, Star, ID). PEG-rhTSH conjugates were serially diluted 1:5 from 16µM to 41pM in assay buffer (100mM HEPES pH 7.5, 20mM EDTA, 1% BSA, 0.5% Triton X-100) and mixed 1:1 with biotinylated Thyrogen^{®} diluted 1000-fold in assay buffer. The mixture was added to each receptor-coated well and incubated at 25°C for 25 minutes. Unbound rhTSH was washed away and streptavidin peroxidase was added at 25°C for 20 minutes according to the RSR Limited ELISA protocol to determine the amount of biotinylated Thyrogen^{®} bound to the plate. The plate was then washed three times to remove excess unbound streptavidin peroxidase and then tetramethylbenzidine (TMB) was added to each well and incubated in the dark at 25°C for 30 minutes. The reaction which was quenched with 0.5M sulfuric acid stop buffer and the absorbance of each well was read at 450 nm using a SpectraMax^{®} 340pc plate reader (Molecular Devices, Sunnyvale, CA). The data was fit using a sigmoidal dose response equation with GraphPad Prism software to generate IC₅₀ values.

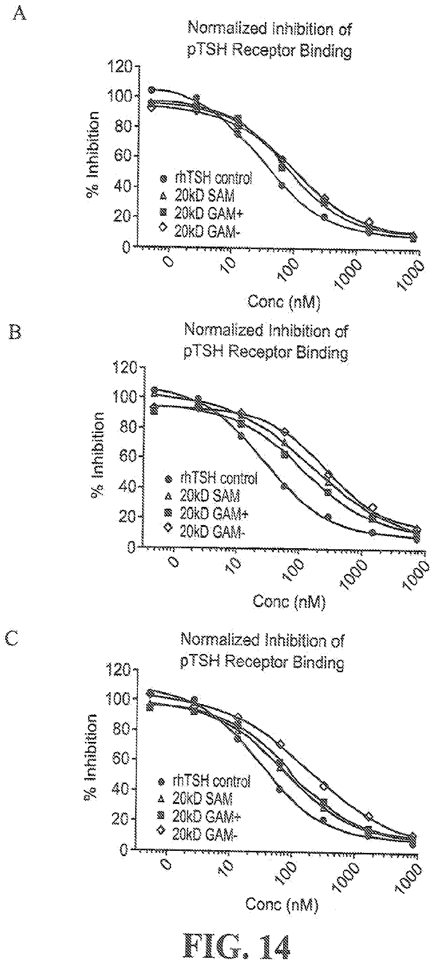

N-terminus and lysine PEGylation yielded conjugates with lower TSH receptor affinity than carbohydrate conjugation. N-terminal mono-PEGylation with 40kD PEG resulted in 10.8-fold lower receptor binding affinity compared to the TSH control. Lysine-PEGylation with 40kD PEG resulted in 31.2-fold lower receptor binding affinity compared to the TSH control. In contrast, GAM+ mono-PEGylation resulted in 2.2-fold lower receptor binding affinity for 20kD PEG conjugation and 3.6-fold lower receptor binding affinity for 40kD PEG conjugation. SAM mono-PEGylation also resulted in moderate decrease in *in vitro* TSH receptor binding affinity compared to N-terminal PEGylation, ranging from 2.1- to 5.3-fold decrease, depending on the size of PEG conjugated. GAM(-) mono-PEGylation caused the greatest loss among all the carbohydrate PEGylation strategies, with 20kD PEG conjugation causing 2.7-fold decrease and 40kD PEG conjugation, 8.0-fold decrease in *in vitro* TSH receptor binding affinity. (See Tables 3a and 3b and FIG. 14).

**Table 3a**

| **Sample** | **Hillslope** | **R²** | **IC₅₀ (nM)** | **fold change** |
|---|---|---|---|---|
| rhTSH | -1.000 | 0.987 | **36.7** | 1.0 |
| 10kD multiSAM | -0.852 | 0.988 | **137.7** | 3.8 |
| 10kD monoSAM | -0.801 | 0.990 | **78.1** | 2.1 |
| 20kD monoSAM | -0.767 | 0.993 | **92.3** | 2.5 |
| 40kD monoSAM | -0.744 | 0.974 | **195.7** | 5.3 |
| 20kD GAM+ | -0.949 | 0.977 | **81.7** | 2.2 |
| 40kD GAM+ | -0.798 | 0.989 | **132.6** | 3.6 |
| 20kD GAM- | -0.690 | 0.996 | **100.6** | 2.7 |
| 40kD GAM- | -0.745 | 0.988 | **293.6** | 8.0 |
| 40kD N-terminal | -0.606 | 0.977 | **397.4** | 10.8 |
| 40kD Lysine | -0.605 | 0.936 | **876.1** | 31.2 |

**Table 3b**

| **Sample** | **Hillslope** | **R²** | **IC₅₀ (nM)** | **fold change** |
|---|---|---|---|---|
| rhTSH | -0.769 | 0.950 | **28.0** | 1.0 |
| G22C | -0.817 | 0.966 | **41.6** | 1.5 |
| 40kD linear G22C | -0.737 | 0.960 | **248.2** | 8.9 |
| 40kD 2-arm PEG G22C | -0.725 | 0.928 | **417.3** | 14.9 |
| 40kD 4-arm PEG G22C | -0.508 | 0.963 | **743.2** | 26.5 |
| 50kD 3-arm PEG G22C | -1.074 | 0.903 | **280.9** | 10.0 |
| 60kD 2-arm PEG G22C | -0.316 | 0.890 | **11342** | 404.5 |
| 60kD 4-arm PEG G22C | -0.274 | 0.906 | **4025000** | 143544 |
| 40kD Lysine | -0.605 | 0.936 | **876.1** | 31.2 |
| 40kD N-terminal | -0.573 | 0.935 | **319.1** | 11.4 |

### Example 13. Pharmacokinetic and Pharmacodynamic Analysis of PEGylated rhTSH in Male and Female Sprague Dawley Rats Following a Single Intramuscular (IM) Injection

The pharmacokinetics of rhTSH and PEGylated rhTSH (20kD SAM, 20kD GAM(-), 20kD GAM(+), 40kD GAM(+)) was evaluated in male and female rats following a single intramuscular (IM) injection.

A single dose of rhTSH or PEGylated rhTSH (20kD SAM, 20kD GAM(-), 20kD GAM(+), 40kD GAM(+)) was administered IM to fasted male and female jugular vein cannulated rats at a dose of 0.5 mg/kg. Due to dose volume limitations, animals received test articles in the form of two or three intramuscular injections into quadriceps muscle. Legs were alternated for dosing. Blood samples were collected from the animals pre-dose and at the following post-dosage time points: 0.5, 1, 2, 4, 8, 24, and 48 hours. Food was removed from the animal cages on the evening prior to test article administration. Animals had access to water during this time. Food was added back to cages following pre-dose sample collections and test article administration. Food was removed again at the end of the day such that animals were fasted for the 24 hour post-dose sample collection. Food was added back to cages following sample collection and removed again at the end of the day such that animals were fasted for the 48 hour post-dose sample collection. Blood was collected from the single port jugular cannula. Approximately 400 µl of whole blood was collected into serum separator tubes and processed for serum. The serum was separated into two tubes (~100 µl each). All samples were stored at -80°C until they were analyzed for rhTSH or PEGylated rhTSH concentration by TSH ELISA. Following the last sample collection animals were euthanized with CO₂.

**Table 4**

| **Group** | **AnimaI #'s** | **Test Article** | **Dose (mg/kg )** | **Dose Route** | **Time Points** |
|---|---|---|---|---|---|
| 1 | 1 - 6 | rhTSH | 0.5 | IM | Pre-dose, 0.5, 1, 2, 4, 8, 24, and 48 hours post dose |
| 2 | 7-12 | PEG rhTSH (20KD SAM) | 0.5 | IM | Pre-dose, 6, 24, 48 and 72 hours post dose |
| 3 | 13-18 | PEG rhTSH (20KD GAM -) | 0.5 | IM | Pre-dose, 6, 24, 48 and 72 hours post dose |
| 4 | 19-24 | PEG rhTSH (20KD GAM +) | 0.5 | IM | Pre-dose, 6, 24, 48 and 72 hours post dose |
| 5 | 25-30 | PEG rhTSH (40KD GAM+) | 0.5 | IM | Pre-dose, 6, 24, 48 and 72 hours post dose |

**Table 5**

| | **rhTSH (n=5)** | **20 KD SAM (n=6)** | **20 KD GAM (-) (n=6)** | **20 KD GAM (+) (n=6)** | **40 KD GAM (+) (n=6)** |
|---|---|---|---|---|---|
| **t_{1/2} (hr)** | 5.68 ± 2.33 | 30.1 ± 6.47 | 27.0 ± 3.91 | 12.6 ± 3.45 | 38.6 ± 19.6 |
| **Cl (ml/hr/kg)** | 131 ± 27.7 | 4.74 ± 0.54 | 3.31 ± 0.41 | 156 ± 25.5 | 12.2 ± 1.88 |
| **Vz (ml/kg)** | 1051 ± 386 | 203 ± 38.8 | 128 ± 22.7 | 2818 ± 843 | 650 ± 233 |
| **Cₘₐₓ (ug/ml)** | 0.42 ± 0.14 | 2.09 ± 0.56 | 3.85 ± 0.79 | 0.23 ± 0.06 | 0.81 ± 0.18 |
| **Tₘₐₓ (hr)** | 2.00 ± 0.00 | 16.0 ± 8.76 | 5.33 ± 3.01 | 1.50 ± 0.55 | 7.00 ± 8.65 |
| **AUCINF (ug*hr/ml)** | 3.94 ± 0.73 | 107 ± 11.2 | 153 ± 21.3 | 3.30 ± 0.65 | 41.6 ± 6.00 |

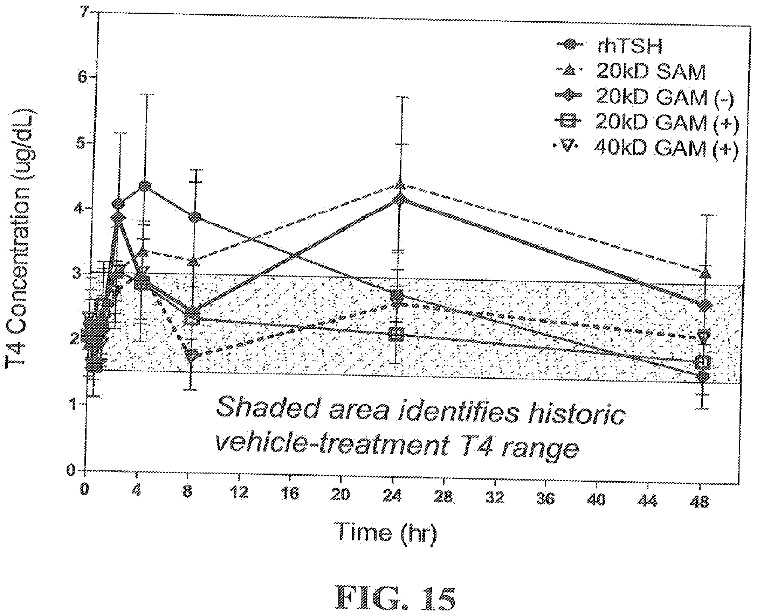

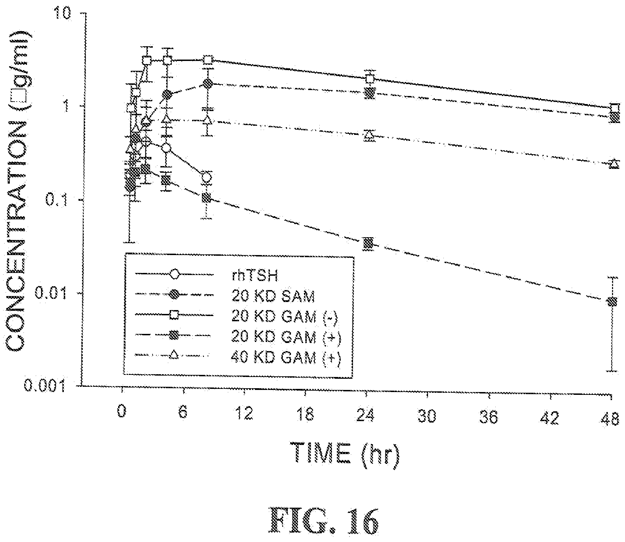

The PK data showed that 20 kD SAM and 20 kD GAM(-) have >5-fold prolonged t1/2, prolonged Tmax and increased exposure (area under the curve, AUC) compared to rhTSH control. The PK profile of 20 kD GAM(+) showed less improvement compared to 20kD SAM and 20kD GAM(-), and 40kD GAM(+) showed only a moderate improvement. Increased value of Vz (apparent volume of distribution) indicated that GAM(+) conjugates may undergo a receptor-mediated clearance. (See FIG. 16 and Table 5). The serum T4 concentration was measured to collect the pharmacodynamic data *(See* FIG. 15), using ACE clinical chemistry system (Alfa Wassermann Diagnostic Technologies, LLC) according to manufacturer's protocol.

### Example 14. Pharmacokinetic Analysis of PEGylated rhTSH in Male and Female Sprague Dawley Rats Following a Single IM Injection

The pharmacokinetics of PEGylated rhTSH (10kD multiSAM, 10kD SAM, 40kD SAM, 40kD GAM(-)) was evaluated in male and female rats following a single intramuscular (IM) injection.

A single dose of rhTSH or PEGylated rhTSH (10kD multiSAM, 10kD SAM, 40kD SAM, 40kD GAM(-)) was administered IM to fasted male and female jugular vein cannulated rats at a dose of 0.5 mg/kg. Due to dose volume limitations, animals received test articles in the form of two or three intramuscular injections into quadriceps muscle. Legs were alternated for dosing. Blood samples were from the animals pre-dose and at the following post-dosage time points: 0.5, 1, 3, 6, 24, 48, 72, and 96 hours. Food was removed from the animal cages on the evening prior to test article administration. Animals had access to water during this time. Food was added back to cages following pre-dose sample collections and test article administration. Food was removed again at the end of each day such that animals were fasted for the post-dose sample collection in the following mornings. Food was added back to cages after each post-dose sample collection. Blood was collected from the single port jugular cannula. Approximately 400 µl of whole blood was collected into serum separator tubes and processed for serum. The serum was separated into two tubes (~100 µl each). All samples were stored at -80°C until they were analyzed for rhTSH or PEGylated rhTSH concentration by TSH ELISA. Following the last sample collection animals were euthanized with CO₂.

**Table 6**

| **Grou p** | **Anima l #'s** | **Test Article** | **Dose (mg/kg )** | **Dose Route** | **Time Points** |
|---|---|---|---|---|---|
| 1 | 1 - 6 | rhTSH | 0.5 | IM | Pre-dose, 0.5, 1, 3, 6, 24, 48, 72, and 96 hours post dose |
| 2 | 7-12 | PEG rhTSH (10KD MultiSAM) | 0.5 | IM | Pre-dose, 0.5, 1, 3, 6, 24, 48, 72, and 96 hours post dose |
| 3 | 13-18 | PEG rhTSH (10KD SAM) | 0.5 | IM | Pre-dose, 0.5, 1, 3, 6, 24, 48, 72, and 96 hours post dose |
| 4 | 19-24 | PEG rhTSH (40KD SAM) | 0.5 | IM | Pre-dose, 0.5, 1, 3, 6, 24, 48, 72, and 96 hours post dose |
| 5 | 25-30 | PEG rhTSH (40KD GAM-) | 0.5 | IM | Pre-dose, 0.5, 1, 3, 6, 24, 48, 72, and 96 hours post dose |

**Table 7**

| | **rhTSH (n=6 )** | **10 KD SAM (n=5 )** | **10 KD Multi SAM (n=6)** | **40 KD SAM (n=4 )** | **40 KD GAM ( -) (n=6)** |
|---|---|---|---|---|---|
| **t_{1/2} (hr)** | 3.42 ± 0.61 | 15.8 ± 1.23 | 46.8 ± 10.4 | 77.9 ± 16.9 | 80.1 ± 16.1 |
| **Cl (ml/hr/kg)** | 167 ± 31.3 | 15.8 ± 1.38 | 2.35 ± 0.77 | 1.04 ± 0.16 | 1.27 ± 0.15 |
| **Vz (ml/kg)** | 810 ± 129 | 358 ± 18.9 | 159 ± 60.4 | 114 ± 9.24 | 146 ± 25.3 |
| **Cₘₐₓ (ug/ml)** | 0.44 ± 0.11 | 1.0 ± 0.11 | 3.11 ± 1.02 | 3.81 ± 0.31 | 3.19 ± 0.55 |
| **Tₘₐₓ (hr)** | 1.50 ± 1.18 | 5.40 ± 1.34 | 14.5 ± 18.33 | 30.0 ± 12.0 | 28.0 ± 9.8 |
| **AUCINF (uq*hr/ml)** | 3.07 ± 0.53 | 31.8 ± 2.71 | 228 ± 59.3 | 492 ± 82.5 | 397 ± 46.9 |

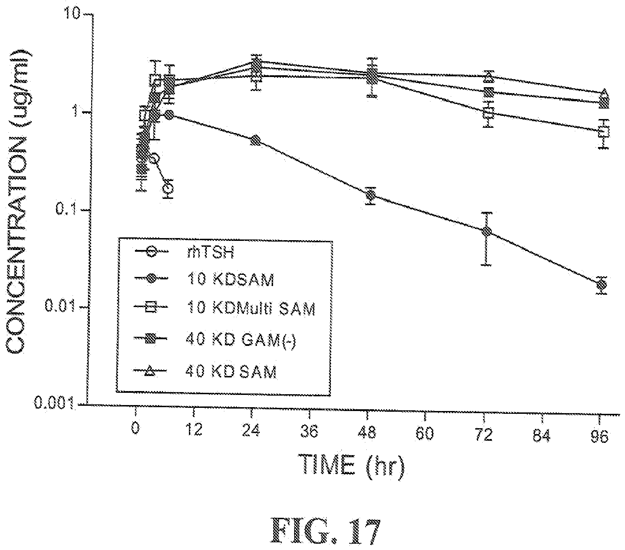

The PK data showed that 10 kD multi-SAM, 40 kD SAM, and 40 kD GAM(-) have 14 ~ 23-fold prolonged t1/2, prolonged Tmax and increased exposure (area under the curve, AUC) compared to rhTSH control. (See FIG. 17 and Table 7) The improvement observed in the PK profiles of 40 kD SAM and 40 kD GAM(-) is greater than that of 20 kD SAM and 20 kD GAM(-) in Example 13.

### In vivo rat pharmacokinetic studies of PEG conjugates

**(Examples 13 and 14):** Overall, plasma half-life was increased proportional to the size of PEG conjugated to rhTSH as expected. For the same PEG-size SAM and GAM(-) conjugates, there was no apparent difference between the two PEGylation strategies for improvement of PK parameters, unlike receptor binding affinity, suggesting that effect on PK is only PEG-size dependent and not PEGylation-site dependent. For example, both 40kD SAM and 40kD GAM(-) showed 23-fold increase in plasma half-life compared to rhTSH control (3.42 ± 0.61 hour) to 77.9 ± 16.9 hours and 80.1 ± 16.1 hours, respectively. MultiPEGylation (10kD multiSAM) had a greater increase of plasma half-life compared to monoPEGylation of the same size PEG (10kD SAM). 10kD multiSAM had 13.7-fold increase to 46.8 ± 10.4 hours compared to rhTSH control whereas 10kD SAM had only 4.6-fold increase to 15.8 ± 1.23 hour. (See Table 7) PEG-size dependent delay in concentration peak time (Tmax) was also observed. For example, 10kD, 20kD and 40kD SAM showed Tmax at 5.40 ± 1.34 hour, 16.0 ± 8.76 hours and 30.0 ± 12.0 hours, respectively, compared to rhTSH control at 2.00 ± 0.00 hour or 1.50 ± 1.18 hour. (See Table 5 and Table 7).

### Example 15. Pharmacokinetic Assay: rhTSH or PEGylated rhTSH ELISA Measuring Protein Concentration in Serum Samples

High binding 96-well ELISA plates were coated with murine anti-hCG capture antibody at 1.33µg/mL diluted in 0.1M sodium bicarbonate buffer at pH 9.2, and added at 100µL per well. A standard rhTSH or PEGylated rhTSH curve was prepared from purified protein and diluted in sample dilution buffer (SDB) consisting of 1.0% w/v BSA in 1X plate wash. The standard was diluted from 25-1.463 ng/mL, 8.334-0.488 ng/mL or 5.556 to 0.325 ng/mL using a 2:3 serial dilution scheme, depending on the qualified linear range of rhTSH or PEGylated rhTSH species per assay. Samples were diluted in SDB at no less than a 1:10 dilution. 500 ng/mL, and 25 ng/mL rhTSH controls prepared in normal rat serum are diluted 1:100 and 1:10 respectively in SDB. A 0.5 ng/mL rhTSH control prepared in SDB was added undiluted. Coated plates were washed and 100µL of samples, standards and controls were added to the plates and incubated for 1 hour at 37°C.

Biotinylated anti-rhTSH monoclonal detection antibody (clone TS8) was diluted in SDB according to the appropriate dilution specific to each lot. Plates were washed and 100µL of the diluted biotinylated detection antibody was added to the wells and incubated for 1 hour at 37°C.

Streptavidin-horseradish peroxidase conjugate (SA-HRP) was diluted in SDB according to the appropriate dilution specific to each lot. Plates were washed and 100µL of the diluted SA-HRP was added to the wells and incubated for 15 minutes at 25°C.

The plates were washed and 100µL of tetramethyl benzidine (TMB) substrate was added to all wells, and incubated for 20 minutes at 25°C.

100µL of TMB stop buffer was added to all wells, and the plate was read at 450nm.

### Example 16. Mouse T4 Bioassay Assessment of PEGylated rhTSH Pharmacodynamics

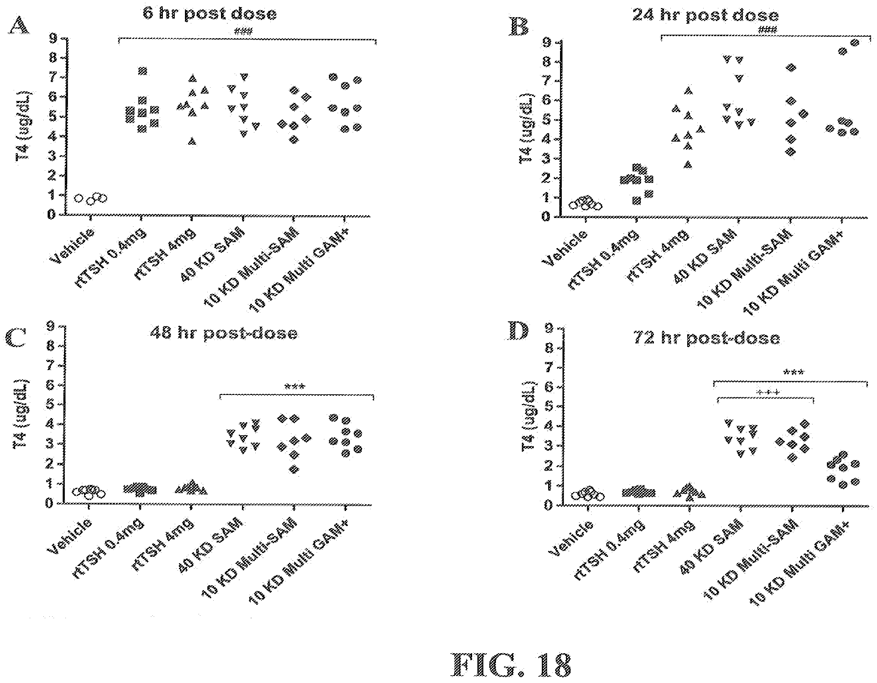

The pharmacodynamics of PEGylated rhTSH was evaluated in male mice following a single IP injection, three days post T3 pellet implantation. In this mouse PD model, the endogenous mouse T4 was suppressed during the study period by implantation of slow-release T3 pellet three days prior to dosing (See vehicle group in FIG. 18A - FIG. 18D). Therefore, only the amount of T4 released by rhTSH control or PEGylated rhTSH conjugates was measured. Due to limited blood volume of mouse, four time points of 6, 24, 48 and 72 hours post-dose were collected.

Animals were anesthetized with isoflurane and a 0.1 mg T3 pellet (T-261, Innovative Research of America) was implanted subcutaneously using a trochar. At three days post pellet implantation, a single dose of rhTSH (0.4 or 4.0 mg/kg) or PEGylated rhTSH (4 mg/kg) was administered IP to male mice (ICR strain, 6 weeks of age, Taconic Farms). Animals were anesthetized with isoflurane and blood samples were collected from the retro-orbital plexus. Group 1 blood samples were collected pre-dose (animals 1-4), 6 (animals 5-8), 24, 48 and 72 hours following test article administration. Blood samples from all other groups were collected at 6, 24, 48, and 72 hours following test article administration. Approximately 60 µl of whole blood was collected into micro-hematocrit capillary tubes and processed for serum. Following the last sample collection, animals were euthanized with CO_{2.} All serum samples were stored at -80°C until they were analyzed for T4 concentrations by the ACE^{®} clinical chemistry T4 assay. Serum T4 concentration was measured by ACE clinical chemistry system (Alfa Wassermann Diagnostic Technologies, LLC) according to manufacturer's protocol.

**Table 8**

| **Grou p** | **Anima l #'s** | **Pellet** | **Dose Route** | **Test Article** | **Dose (mg/kg)** | **Dose Route** | **Time Points** |
|---|---|---|---|---|---|---|---|
| 1 | 1 - 8 | T3, 0.1mg | SC | Vehicle (0.2% BSA in PBS) | 0.0 | IP | Pre-dose (1-4), 6 (5-8), 24, 48, and 72 hours post dose |
| 2 | 9-16 | T3, 0.1mg | SC | rhTSH | 0.4 | IP | 6, 24, 48, and 72 hours post-dose |
| 3 | 17-24 | T3, 0.1mg | SC | rhTSH | 4 | IP | 6, 24, 48, and 72 hours post-dose |
| 4 | 25-32 | T3, 0.1mg | SC | PEG rhTSH 40KD SAM | 4 | IP | 6, 24, 48, and 72 hours post-dose |
| 5 | 33-40 | T3, 0.1mg | SC | PEG rhTSH 10KD MultiSAM | 4 | IP | 6, 24, 48, and 72 hours post-dose |
| 6 | 41-48 | T3, 0.1mg | SC | PEG rhTSH 10KD MultiGAM (+) | 4 | IP | 6, 24, 48, and 72 hours post-dose |

Data from vehicle-treated animals indicated model validity due to T4 suppression for duration of study via subcutaneous T3 pellet. At 6 hours, all treatments significantly (p< 0.001) increased T4 compared to vehicle (FIG.18A). At 24 hours, 0.4 mg/kg rhTSH began to return to vehicle-like levels of T4 whereas the remainder of the treatments remained significantly (p< 0.001) elevated compared to vehicle (FIG.18B). By 48 hours, both (0.4 mg/kg and 4 mg/kg) rhTSH treatments had returned to vehicle-like levels of T4 and the three PEG conjugates had decreased modestly but were significantly (p< 0.001) elevated compared to rhTSH at 4mg/kg (FIG.18C). At 72 hours, 40KD SAM and 10KD multi-SAM remained elevated (similar to 48 hour time point) while 10KD multi-GAM(+) dropped modestly (FIG.18D). All conjugates remained elevated compared to rhTSH 4mg/kg and, additionally, 40 KD SAM and 10KD multi-SAM were (p< 0.001) elevated compared to 10KD Multi-GAM(+).

10 KD Multi SAM and 40 KD SAM appeared to be promising candidates based on the PK (Example 14) and PD (Example 16) data. 10 KD Multi-GAM(+) appeared promising as well, but did not have as much improved duration of action compared to the two more promising candidates.

### Example 17. Dose Response Curve Study Assessment of PEGylated rhTSH Pharmacodynamics (40kD SAM)

The pharmacodynamics of PEGylated rhTSH (40kD SAM) was evaluated at three different dose levels in male mice following a single intraperitoneal (IP) injection, three days post T3 pellet implantation.

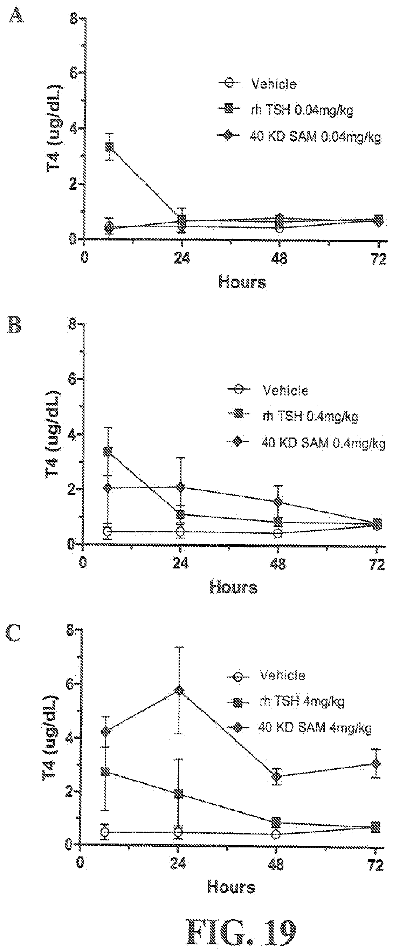

Animals were anesthetized with isoflurane and a 0.1 mg T3 pellet (T-261, Innovative Research of America) was implanted subcutaneously using a trochar. At three days post pellet implantation a single dose of rhTSH or PEGylated rhTSH (40kD SAM) was administered IP to male mice (ICR strain, 6 weeks of age, Taconic Farms) at a dose of 0.04 mg/kg (FIG. 19A), 0.4 mg/kg (FIG. 19B), or 4 mg/kg (FIG. 19C). Animals were anesthetized with isoflurane and blood samples were collected from the retro-orbital plexus. Group 1 blood samples were collected 6 (animals 1-4), 24, 48, 72 and 96 (animals 5-8) hours following test article administration. Blood samples from all other groups were collected at 6, 24, 48, and 72 hours following test article administration. Approximately 60 µl of whole blood was collected into micro-hematocrit capillary tubes and processed for serum. Following the last sample collection, animals were euthanized with CO₂. All serum samples were stored at -80°C until they were analyzed for T4 concentrations by the ACE^{®} clinical chemistry T4 assay. Serum T4 concentration was measured by ACE clinical chemistry system (Alfa Wassermann Diagnostic Technologies, LLC) according to manufacturer's protocol.

**Table 9**

| **Grou p** | **Animal #'s** | **Pellet** | **Dose Route** | **Test Article** | **Dose (mg/kg )** | **Dose Route** |
|---|---|---|---|---|---|---|
| 1 | 1 - 8 | T3, 0.1mg | SC | Vehicle (0.2% BSA in PBS) | 0.0 | IP |
| 2 | 9-16 | T3, 0.1mg | SC | rhTSH | 0.04 | IP |
| 3 | 17-24 | T3, 0.1mg | SC | rhTSH | 0.4 | IP |
| 4 | 25-32 | T3, 0.1mg | SC | rhTSH | 4 | IP |
| 5 | 33-40 | T3, 0.1mg | SC | PEG rhTSH 40KD SAM | 0.04 | IP |
| 6 | 41-48 | T3, 0.1mg | SC | PEG rhTSH 40KD SAM | 0.4 | IP |
| 7 | 49-56 | T3, 0.1mg | SC | PEG rhTSH 40KD SAM | 4 | IP |

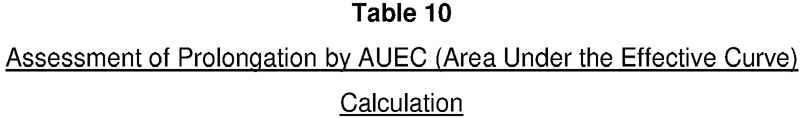

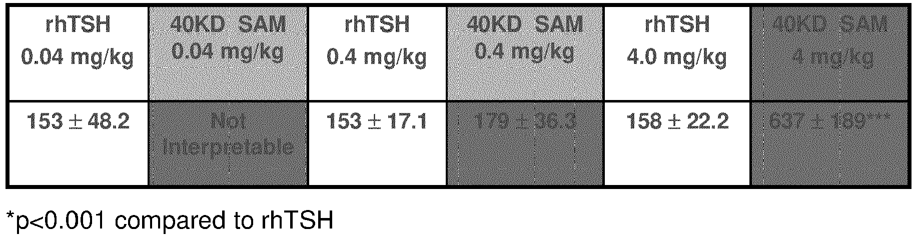

Data from vehicle-treated animals indicated model validity due to T4 suppression for duration of study via subcutaneous T3 pellet. All doses of rhTSH showed equivalent AUEC and elicited an apparent maximal effect at the top of the dose response curve. 40 KD SAM exhibited AUEC representing a full dose response curve, from no effect, to modest effect, to apparent maximal effect. The rhTSH treatment increased T4 levels at all doses at 6 hours and then decreased to the vehicle-like levels of T4 at 24 hours except for the highest dose, 4 mg/kg, which showed T4 levels returning to vehicle levels at 48 hours post-dose. Prolongation of effect with 40 KD SAM was dependent upon dose in this mouse PD model, most significant at 4 mg/kg dose level. Only a mild enhancement of effect was seen with 0.4 mg/kg level.

### Example 18. Pharmacodynamic Analysis of PEGylated rhTSH in Male and Female Sprague Dawley Rats Following a Single IM Injection, Three Days Post T3 Pellet Implantation

The pharmacodynamics of PEGylated rhTSH was evaluated in male and female rats following a single intramuscular (IM) injection, three days post T3 pellet implantation.

Animals were anesthetized with isoflurane and a 1.5 mg T3 pellet (T-261, Innovative Research of America) was implanted subcutaneously using a trochar. At three days post pellet implantation, a single dose of rhTSH or PEGylated rhTSH was administered IM to male and female jugular vein cannulated rats at a dose of 0.0 mg/kg (vehicle), 0.04 mg/kg, 0.4 mg/kg, or 0.65 mg/kg. For 40kD multiSAM and 50kD multiSAM, 0.65 mg/kg dose was determined based on the content of monoPEGylated species. Due to dose volume limitations, animals received test articles in the form of two intramuscular injections into the quadriceps muscle. Legs were alternated for dosing. Blood samples were collected from the animals pre-dose and at the following post-dosage time points: 1, 3, 6, 24, 48, 72, 96, 120, 144, and 168 hours. Blood was collected from the single port jugular cannula. Approximately 250 µl of whole blood was collected into serum separator tubes and the blood was allowed to clot for a minimum of 30 minutes. Tubes were spun in a centrifuge at 10,000 rpm for 5 minutes and the serum was separated into two tubes (~50 µl each). All samples were stored at -80°C until they were analyzed for T4 concentrations by the ACE^{®} clinical chemistry T4 assay. Following the last sample collection animals were euthanized with CO₂.Serum T4 concentration was measured by ACE clinical chemistry system (Alfa Wassermann Diagnostic Technologies, LLC) according to manufacturer's protocol.

**Table 11**

| **Grou p** | **Anima I #'s Male** | **Anima I #'s Femal** e | **Pellet** | **Dose Route** | **Test Article** | **Dose (mg/kg) /Route** |
|---|---|---|---|---|---|---|
| 1 | 1-2 | 3-4 | T3, 1.5mg | SC | Vehicle (0.2% BSA in PBS) | 0.0/IM |
| 2 | 5-6 | 7-8 | T3, 1.5mg | SC | rhTSH | 0.04/IM |
| 3 | 9-10 | 11-12 | T3, 1.5mg | SC | rhTSH | 0.4/IM |
| 4 | 13-15 | 16-18 | T3, 1.5mg | SC | PEG rhTSH 10KD MultiSAM | 0.04/IM |
| 5 | 19-21 | 22-24 | T3, 1.5mg | SC | PEG rhTSH 10KD MultiSAM | 0.4/IM |
| 6 | 25-27 | 29-30 | T3, 1.5mg | SC | PEG rhTSH 40KD SAM | 0.4/IM |
| 7 | 31-33 | 33-36 | T3, 1.5mg | SC | PEG rhTSH 40KD MultiSAM | 0.65/IM |
| 8 | 37-39 | 40-42 | T3, 1.5mg | SC | PEG rhTSH 50KD MultiSAM | 0.65/IM |

**Table 12**

| Assessment of Prolongation by AUEC (Area Under the Effective Curve) Calculation | | | | | | | |
|---|---|---|---|---|---|---|---|
| AUEC Calculation | rhTSH 0.04 mg/kg | rhTSH 0.4 mg/kg | 10KD Multi 0.04 mg/kg | 10KD Multi 0.4 mg/kg | 40KD SAM 0.4 mg/kg | 40KD Multi 0.65 mg/kg | 50KD Multi 0.65 mg/kg |
| 0-96 hrs | 113 ± 39.1 | 151 ± 34.7 | 62.2 ± 33.2 | 200 ± 77.6 | 227 ± 72.0 | 261 ± 49.6 | 294 ± 86.9* |
| 0-120 hrs | 119 ± 44.5 | 151 ± 34.7 | 62.2 ± 33.2 | 201 ± 75.9 | 237 ± 78.2 | 271 ± 54.1 | 319 ± 102* |
| 0-144 hrs | 129 ± 49.8 | 151 ± 34.7 | 62.2 ± 33.2 | 201 ± 75.9 | 240 ± 79.3 | 272 ± 55.1 | 327 ± 110* |
| 0-168 hrs | 143.8 ± 64.4 | 152 ± 32.0 | 64.6 ± 32.6 | 203 ± 76.7 | 243 ± 79.8 | 275 ± 56.1 | 333 ± 110* |

| | | | | | | | |
|---|---|---|---|---|---|---|---|
| *p<0.05 compared to rhTSH | | | | | | | |

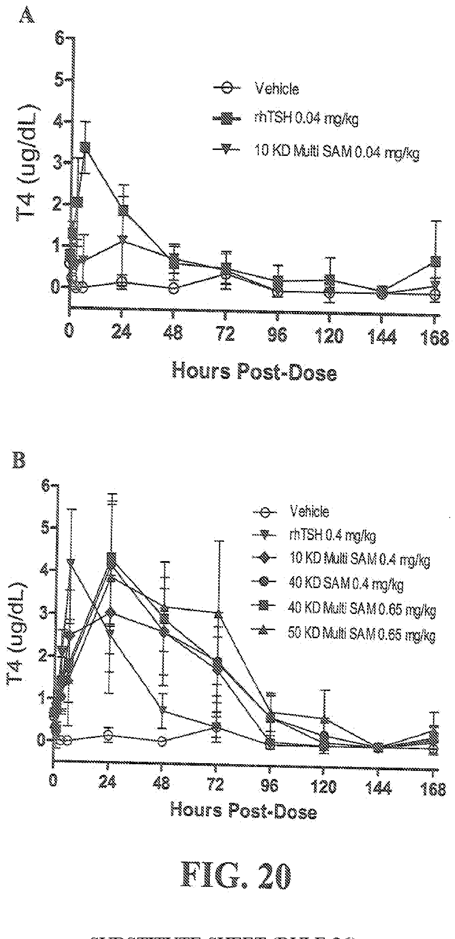

Data from vehicle-treated animals indicated model validity due to T4 suppression for duration of study via subcutaneous T3 pellet. The Tmax of rhTSH occurred at 6 hours post-dose and returned to baseline *(i.e.,* vehicle group levels) by 72 hour post-dose. 10 KD multiSAM Tmax occurred at 24 hours post-dose and returned to baseline at 72 hours post-dose for 0.04 mg/kg dose and 96 hours post-dose for 0.4 mg/kg dose. Low dose data showed decreased potency of 10 KD multiSAM relative to rhTSH in this model, which may reflect decreased TSH receptor binding affinity. High dose data confirmed enhanced pharmacodynamic effects with 10 KD multiSAM at 48 and 72 hours post-dose. All candidates including 40kD multiSAM and 50kD multiSAM showed enhanced T4 response compared to rhTSH at 0.4 mg/kg, which might have been in part mediated by shift in Tmax to 24 hours post-dose. Prolonged pharmacodynamic activity was observed through 96-120 hours post-dose. (For 40kD multiSAM and 50kD multiSAM, 0.65 mg/kg dose equaled to 0.4 mg/kg based on the percentage content of monoPEGylated species in these conjugates.) For all test articles, approximately 95% of the pharmacodynamic effects were observed within 96 hours post-dose following intramuscular administration. Higher AUECs observed with PEGylated conjugates is driven by higher T4 levels between 24-96 hours post-dose. For the 0-96 hour period, 10 KD multiSAM exhibited 1.3-fold of rhTSH AUEC, 40 KD SAM, 1.5-fold, 40 KD multiSAM, 1.7-fold, and 50 KD multiSAM, 1.9-fold. (Table 12, FIG. 20).

### Example 19. Pharmacodynamic Analysis of PEGylated rhTSH in Male and Female Sprague Dawley Rats Following a Single IM Injection, Three Days Post T3 Pellet Implantation

The pharmacodynamics of PEGylated rhTSH was evaluated in male and female rats following a single intramuscular (IM) injection, three days post T3 pellet implantation.

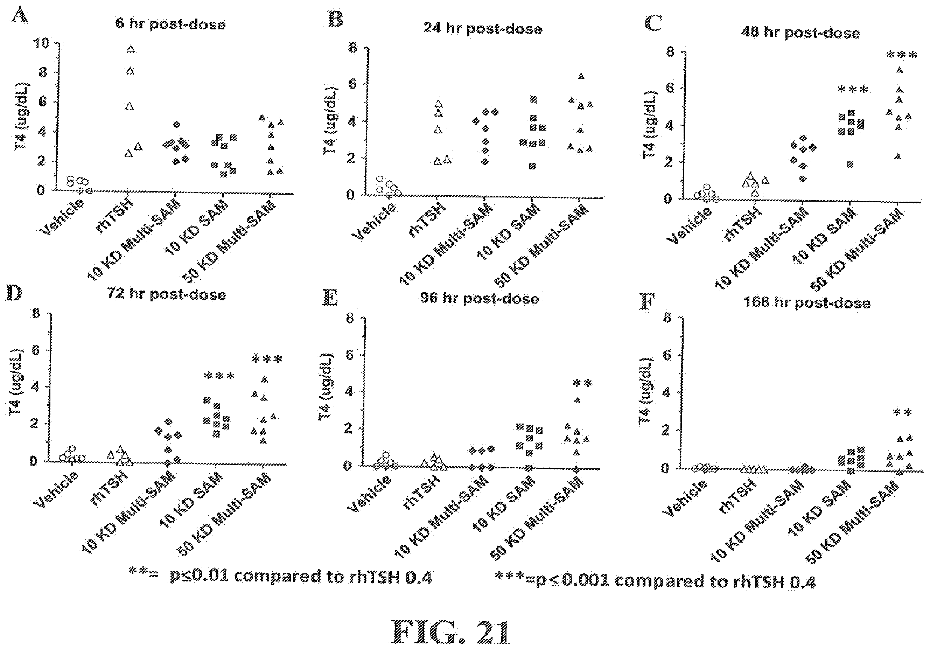

Animals were anesthetized with isoflurane and a 1.5 mg T3 pellet (T-261, Innovative Research of America) was implanted subcutaneously using a trochar. At three days post pellet implantation, a single dose of rhTSH or PEGylated rhTSH was administered IM to male and female jugular vein cannulated rats at a dose of 0.0 mg/kg (vehicle), 0.4 mg/kg, or 0.65 mg/kg. For 50kD multiSAM, 0.65 mg/kg dose was determined based on the content of monoPEGylated species. Due to dose volume limitations, animals received test articles in the form of two intramuscular injections into the quadriceps muscle. Legs were alternated for dosing. Blood samples were collected from the animals pre-dose and at the following post-dosage time points: 6 (FIG.21A), 24 (FIG.21B), 48 (FIG.21C), 72 (FIG.21D), 96 (FIG.21E), and 168 (FIG.21F) hours. Blood was collected from the single port jugular cannula. Approximately 250 µl of whole blood was collected into serum separator tubes and the blood was allowed to clot for a minimum of 30 minutes. Tubes were spun in a centrifuge at 10,000 rpm for 5 minutes and the serum was separated into two tubes (~50 µl each). All samples were stored at -80°C until they were analyzed for T4 concentrations by the ACE^{®} clinical chemistry T4 assay. Following the last sample collection animals were euthanized with CO₂. Serum T4 concentration was measured by ACE clinical chemistry system (Alfa Wassermann Diagnostic Technologies, LLC) according to manufacturer's protocol.

**Table 13**

| **Grou p** | **Anima I #'s Male** | **Anima I #'s Femal e** | **Pellet** | **Dose Route** | **Test Article** | **Dose (mg/kg) /Route** |
|---|---|---|---|---|---|---|
| 1 | 1-3 | 4-6 | T3, 1.5mg | SC | Vehicle (0.2% BSA in PBS) | 0.0/IM |
| 2 | 7-9 | 10-12 | T3, 1.5mg | SC | rhTSH | 0.4/IM |
| 3 | 13-16 | 17-20 | T3, 1.5mg | SC | PEG rhTSH 10KD MultiSAM | 0.4/IM |
| 4 | 21-24 | 25-28 | T3, 1.5mg | SC | PEG rhTSH 40KD SAM | 0.4/IM |
| 5 | 29-32 | 33-36 | T3, 1.5mg | SC | PEG rhTSH 50KD MultiSAM | 0.65/IM |

**Table 14**

| Assessment of Prolongation bv AUEC (Area Under the Effective Curve) Calculation | | | | |
|---|---|---|---|---|
| AUEC Calculation | rhTSH 0.4 mg/kg | 10KD Multi 0.4 mg/kg | 40KD SAM 0.4 mg/kg | 50KD Multi 0.4 mg/kg |
| 0-96 Hours | 176 ± 82 | 203.7 ± 62.8 | 277 ± 68 | 337 ± 111 |
| 0-168 Hours | 190 ± 87.7 | 245 ± 82.6 | 429 ± 82.0 | 506 ± 231 |

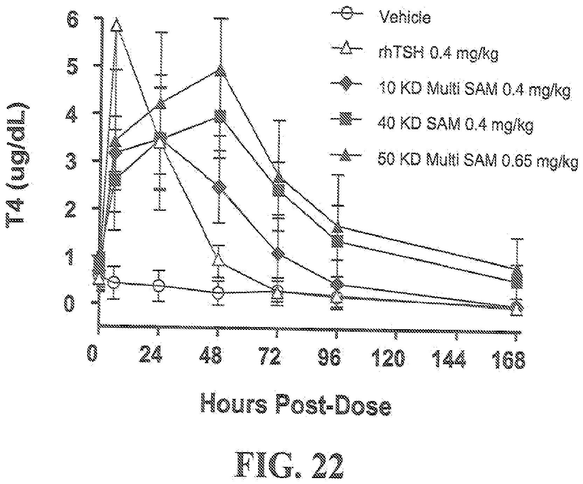

Data from vehicle-treated animals indicated model validity due to T4 suppression for duration of study via subcutaneous T3 pellet. (See FIG. 22). Prolonged pharmacodynamic effects were consistent within the group for each test article. Both the duration of action and AUEC ranked from rhTSH < 10 KD multiSAM < 40 KD SAM ≤ 50 KD multiSAM, consistent with Example18.

### Example 20. Pharmacodynamic Assessment of PEGylated rhTSH (10KD MultiSAM and 40KD SAM)

The pharmacodynamics of PEGylated rhTSH (10KD MultiSAM and 40KD SAM) was evaluated in male and female rats following a single intramuscular (IM) injection, three days post T3 pellet implantation.

Animals were anesthetized with isoflurane and a 1.5 mg T3 pellet (T-261, Innovative Research of America) was implanted subcutaneously using a trochar. At three days post pellet implantation, a single dose of vehicle or PEGylated rhTSH (10KD MultiSAM or 40KD SAM) was administered IM to male and female jugular vein cannulated rats at a dose of 0.0 mg/kg (vehicle), 0.1, 0.2, 0.4, or 1.0 mg/kg as specified in Table 15. Due to dose volume limitations, animals received test articles in the form of two intramuscular injections into the quadriceps muscle. Legs were alternated for dosing. Blood samples were collected from the animals pre-dose and at the following post-dosage time points: 6, 24, 48, 72, 96, and 168 hours. Blood was collected from the single port jugular cannula. Approximately 250 µl of whole blood was collected into serum separator tubes and the blood was allowed to clot for a minimum of 30 minutes. Tubes were spun in a centrifuge at 10,000 rpm for 5 minutes and the serum was separated into two tubes (~50 µl each). All samples were stored at -80°C until they were analyzed for T4 concentrations by the ACE clinical chemistry T4 assay. Following the last sample collection animals were euthanized with CO₂. Serum T4 concentration was measured by ACE clinical chemistry system (Alfa Wassermann Diagnostic Technologies, LLC) according to manufacturer's protocol.

**Table 15**

| **Grou p** | **Anima l #'s Male** | **Anima l #'s Femal** e | **Pellet** | **Dose Route** | **Test Article** | **Dose** (**mg/kg) /Route** |
|---|---|---|---|---|---|---|
| 1 | 1-2 | 3-4 | T3, 1.5mg | SC | Vehicle (0.2% BSA in PBS) | 0.0/IM |
| 2 | 5-6 | 7-9 | T3, 1.5mg | SC | PEG rhTSH 10KD MultiSAM | 0.2/IM |
| 3 | 10-11 | 12-14 | T3, 1.5mg | SC | PEG rhTSH 10KD MultiSAM | 0.4/IM |
| 4 | 15-16 | 17-19 | T3, 1.5mg | SC | PEG rhTSH 10KD MultiSAM | 1.0/IM |
| 5 | 20-22 | 23-24 | T3, 1.5mg | SC | PEG rhTSH 40KD SAM | 0.1/IM |
| 6 | 25-27 | 28-29 | T3, 1.5mg | SC | PEG rhTSH 40KD SAM | 0.2/IM |
| 7 | 30-32 | 33-34 | T3, 1.5mg | SC | PEG rhTSH 40KD SAM | 0.4/IM |

**Table 16**

| Assessment of Prolongation by AUEC (Area Under the Effective Curve) Calculation | | | | | | |
|---|---|---|---|---|---|---|
| AUEC Calculation | 10KD Multi 0.2 mg/kg | 10KD Multi 0.4 mg/kg | 10KD Multi 1 mg/kg | 40KD SAM 0.1 mg/kg | 40KD SAM 0.2 mg/kg | 40KD SAM 0.4 mg/kg |
| 0-168 Hours | 177 ± 33.8 | 213 ± 81.8 | 340 ± 117 | 75.6 ± 3.41 | 174 ± 115 | 397 ± 68.1 |

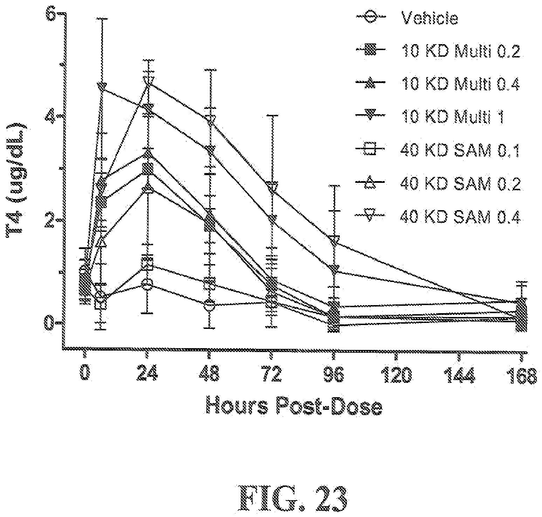

Data from vehicle-treated animals indicated model validity due to T4 suppression for duration of study via subcutaneous T3 pellet. (See FIG. 23). 10 KD multiSAM did not produce a maximum PD response at 0.4 mg/kg and 1 mg/kg resulted in more pronounced T4 responses (in degree and duration). 40 KD SAM 0.2 mg/kg exhibited a moderate PD response while 0.1 mg/kg had little or no effect. Interestingly, 10 KD multiSAM and 40 KD SAM exhibited nearly identical PD at 0.2 mg/kg. At 0.4 mg/kg, however, 40kD SAM elicited a stronger pharmacodynamic effect than 10kD multiSAM. Overall, the data from three separate studies (Examples 18, 19 and 20) are consistent for 10 kD multiSAM and 40 kD SAM.

### Example 21. Pharmacodynamic (PD) Evaluation of PEGylated Cys-Mutant TSH (40KD G22C) Compared to Two Dose Levels of PEGylated rhTSH (40KD SAM) in Male and Female Sprague Dawley Rats Following a Single IM Injection, Three Days Post T3 Pellet Implantation

The pharmacodynamics of PEGylated Cys-Mutant TSH (40KD G22C) was evaluated and compared to the pharmacodynamics of rhTSH and 40kD SAM PEGylated rhTSH in T3 pellet implanted Sprague Dawley rats.

Animals were anesthetized with isoflurane and a 1.5 mg T3 pellet (T-261, Innovative Research of America) was implanted subcutaneously using a trochar. At three days post pellet implantation, a single dose of vehicle, rhTSH, PEGylated rhTSH (40KD SAM), or PEGylated Cys-mutant TSH (40KD G22C) was administered IM to male and female jugular vein cannulated rats at a dose of 0.0 mg/kg (vehicle), 0.2 or 0.4 mg/kg. Due to dose volume limitations, animals received test articles in the form of two intramuscular injections into the quadriceps muscle. Legs were alternated for dosing. Blood samples were collected from the animals pre-dose and at the following post-dosage time points: 6, 24, 48, 72, 96, and 168 hours. Blood was collected from the single port jugular cannula. Approximately 250 µl of whole blood was collected into serum separator tubes and the blood was allowed to clot for a minimum of 30 minutes. Tubes were spun in a centrifuge at 10,000 rpm for 5 minutes and the serum was separated into two tubes (~50 µl each). All samples were stored at -80°C until they were analyzed for T4 concentrations by the ACE clinical chemistry T4 assay. Following the last sample collection animals were euthanized with CO₂. Serum T4 concentration was measured by ACE clinical chemistry system (Alfa Wassermann Diagnostic Technologies, LLC) according to manufacturer's protocol.

**Table 17**

| **Grou p** | **Anima l #'s Male** | **Anima l #'s Femal e** | **Pellet** | **Dose Route** | **Test Article** | **Dose (mg/kg) /Route** |
|---|---|---|---|---|---|---|
| 1 | 1-2 | 3 | T3, 1.5mg | SC | Vehicle (0.2% BSA in PBS) | 0.0/IM |
| 2 | 4 | 5-6 | T3, 1.5mg | SC | rhTSH | 0.4/IM |
| 3 | 7-9 | 10-12 | T3, 1.5mg | SC | PEGylated rhTSH (40KD SAM) | 0.2/IM |
| 4 | 13-15 | 16-18 | T3, 1.5mg | SC | PEGylated rhTSH (40KD SAM) | 0.4/IM |
| 5 | 19-21 | 22-24 | T3, 1.5mg | SC | PEGylated Cys-mutant TSH (40KD G22C) | 0.4/IM |

**Table 18**

| Assessment of Prolongation by AUEC (Area Under the Effective Curve) Calculation | | | | |
|---|---|---|---|---|
| AUEC Calculation(hr*µg/dL) | rhTSH 0.4 mg/kg | 40 KD SAM 0.2 mg/kg | 40 KD SAM 0.4 mg/kg | Cys-Mutant 40kD G22C 0.4 mg/kg |
| 0-168 Hours | 221 ± 90.0 | 276 ± 115 | 319 ± 133 | 212 ± 101 |

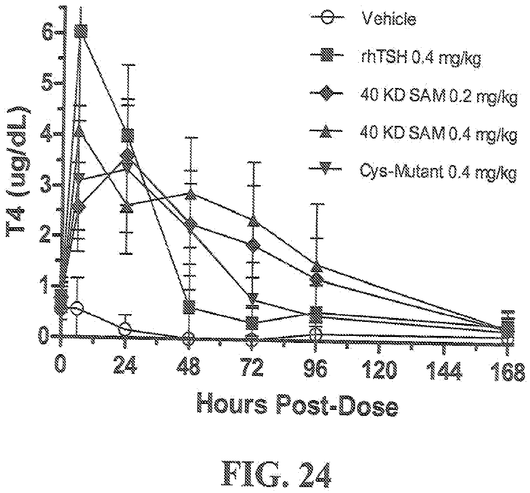

Data from vehicle-treated animals indicated model validity due to T4 suppression for duration of study via subcutaneous T3 pellet. (See FIG. 24) From a pharmacodynamics perspective, the 40 kD G22C Cys-Mutant did not appear to be superior to 40 kD SAM. 40 kD SAM appeared to show increased duration of action compared to the 40 kD G22C Cys-Mutant.

## Claims

1. A composition comprising recombinant human thyroid stimulating hormone (rhTSH), wherein at least one polyalkylene glycol polymer is attached to the rhTSH at:
a) a sialic acid group selected from the group consisting of ASN52 of the rhTSH α subunit, ASN78 of the rhTSH α subunit, and ASN23 of the rhTSH β subunit or a combination thereof; or
b) a galactose located at ASN52 of the rhTSH α subunit, ASN78 of the rhTSH α subunit, ASN23 of the rhTSH β subunit or a combination thereof.

2. The composition of claim 1, where the polyalkylene glycol polymer is polyethylene glycol (PEG).

3. The composition of claim 10, wherein the PEG has an average molecular weight of between about 3,000 and about 100,000 kDa.

4. The composition of claim 3, wherein one PEG is attached to the rhTSH.

5. The composition of claim 3, wherein more than one PEG is attached to the rhTSH.

6. The composition of claim 1 wherein the rhTSH exhibits a prolonged T4 response compared to a control.

7. A composition comprising a mutated recombinant human thyroid stimulating hormone (rhTSH) and at least one polyalkylene glycol polymer, wherein the mutated rhTSH has one or more amino acid residues substituted with a cysteine residue,
wherein the amino acid residue that has been substituted with cysteine is located on the alpha subunit of rhTSH at an amino acid position selected from the group consisting of ASN66, THR69, and GLY22, and combinations thereof; or is located on the beta subunit of rhTSH at the amino acid position of VAL118; or any combination thereof; and
wherein the polyalkylene glycol polymer is attached to the mutated rhTSH at the cysteine residue, and the mutated rhTSH is biologically active.

8. The composition of claim 7, where the polyalkylene glycol polymer is polyethylene glycol (PEG).

9. The composition of claim 8, wherein the PEG has an average molecular weight of between about 3,000 and about 100,000 kDa.

10. The composition of claim 8, wherein one PEG is attached to the rhTSH.

11. The composition of claim 8, wherein more than one PEG is attached to the rhTSH.

12. A method of producing a biologically active recombinant human thyroid stimulating hormone (rhTSH) conjugated to at least one polyalkylene glycol polymer, the method comprising attaching at least one polyalkylene glycol polymer to a rhTSH at:
a) a sialic acid group selected from the group consisting of ASN52 of the rhTSH α subunit, ASN78 of the rhTSH α subunit, and ASN23 of the rhTSH β subunit or a combination thereof; or
b) a galactose located at ASN52 of the rhTSH α subunit, ASN78 of the rhTSH α subunit, ASN23 of the rhTSH β subunit or a combination thereof

13. A method of producing a biologically active mutated recombinant human thyroid stimulating hormone (rhTSH) conjugated to at least one polyalkylene glycol polymer, the method comprising
a) introducing one or more additional cysteine residues into rhTSH, thereby producing a mutated rhTSH, wherein the cysteine residue replaces an endogenous amino acid residue located on the alpha subunit of rhTSH at an amino acid position selected from the group consisting of ASN66, THR69, and GLY22, and combinations thereof, or located on the beta subunit of rhTSH at the amino acid position of VAL118, and combinations thereof; and
b) attaching one or more polyalkylene glycol polymers to the one or more cysteine residues introduced in step a), thereby producing a biologically active mutated rhTSH conjugated to at least one polyalkylene glycol polymer.

14. The method of claim 13, wherein the polyalkylene glycol polymer is polyethylene glycol (PEG).

15. The method of claim 14, wherein the PEG has an average molecular weight of between about 3,000 and about 100,000 kDa.

16. The method of claim 14, wherein one PEG is attached to the rhTSH.

17. The method of claim 14, wherein more than one PEG is attached to the rhTSH.

18. A biologically active recombinant human thyroid stimulating hormone (rhTSH) for use for treating a thyroid condition in a subject in need thereof, wherein at least one polyalkylene glycol polymer is attached to the rhTSH at:
a) a sialic acid group selected from the group consisting of ASN52 of the rhTSH α subunit, ASN78 of the rhTSH α subunit, and ASN23 of the rhTSH β subunit or a combination thereof; or
b) a galactose located at ASN52 of the rhTSH α subunit, ASN78 of the rhTSH α subunit, ASN23 of the rhTSH β subunit or a combination thereof.
and wherein an effective amount of the biologically active rhTSH is administered to the patient.

19. The biologically active rhTSH for the use of claim 18, wherein the polyalkylene glycol polymer is polyethylene glycol (PEG).

20. The biologically active rhTSH for the use of claim 19, wherein the PEG has an average molecular weight of between about 3,000 and about 100,000 kDa.

21. The biologically active rhTSH for the use of claim 19, wherein one PEG is attached to the rhTSH.

22. The biologically active rhTSH for the use of claim 19, wherein more than one PEG is attached to the rhTSH.

23. A pharmaceutical composition comprising an effective therapeutic amount of a composition of any one of claims 1-11 and a pharmaceutically acceptable carrier.

24. A pharmaceutical composition of claim 23 for use in the treatment of a thyroid condition in a patient in need thereof, wherein an effective amount of a pharmaceutical composition of claim 23 is administered to the patient.

25. The pharmaceutical composition for the use of claim 24, wherein the pharmaceutical composition exhibits an enhanced T4 response compared to a control.

26. The pharmaceutical composition for the use of claim 24, wherein the thyroid condition is thyroid cancer.

27. The pharmaceutical composition for the use of claim 24, wherein the pharmaceutical composition is delivered via intramuscular injection.

## Patentansprüche

1. Zusammensetzung, umfassend rekombinantes menschliches Thyroidea-stimulierendes Hormon (rhTSH), wobei mindestens ein Polyalkylenglykolpolymer an das rhTSH angelagert ist, an:
a) einer Sialinsäuregruppe, ausgewählt aus der Gruppe bestehend aus ASN52 der rhTSH-α-Untereinheit, ASN78 der rhTSH-α-Untereinheit und ASN23 der rhTSH-ß-Untereinheit oder einer Kombination davon; oder
b) einer Galaktose, die sich an ASN52 der rhTSH α-Untereinheit, ASN78 der rhTSH α-Untereinheit, ASN23 der rhTSH β-Untereinheit oder einer Kombination davon befindet.

2. Zusammensetzung nach Anspruch 1, wobei das Polyalkylenglykolpolymer Polyethylenglykol (PEG) ist.

3. Zusammensetzung nach Anspruch 10, wobei das PEG ein durchschnittliches Molekulargewicht zwischen etwa 3000 und etwa 100.000 kDa aufweist.

4. Zusammensetzung nach Anspruch 3, wobei ein PEG an das rhTSH angelagert ist.

5. Zusammensetzung nach Anspruch 3, wobei mehr als ein PEG an das rhTSH angelagert ist.

6. Zusammensetzung nach Anspruch 1, wobei das rhTSH im Vergleich zu einer Kontrolle eine verlängerte T4-Antwort aufweist.

7. Zusammensetzung, umfassend ein mutiertes rekombinantes menschliches Thyroidea-stimulierendes Hormon (rhTSH) und mindestens ein Polyalkylenglykolpolymer, wobei das mutierte rhTSH einen oder mehrere Aminosäurereste aufweist, die durch einen Cysteinrest substituiert sind,
wobei sich der Aminosäurerest, der durch Cystein substituiert wurde, an der Alpha-Untereinheit von rhTSH an einer Aminosäureposition befindet, die ausgewählt ist aus der Gruppe, bestehend aus ASN66, THR69 und GLY22 und Kombinationen davon; oder sich an der Beta-Untereinheit von rhTSH an der Aminosäureposition von VAL118 befindet; oder eine beliebige Kombination davon; und
wobei das Polyalkylenglykolpolymer an das mutierte rhTSH an dem Cysteinrest angelagert ist und das mutierte rhTSH biologisch aktiv ist.

8. Zusammensetzung nach Anspruch 7, wobei das Polyalkylenglykolpolymer Polyethylenglykol (PEG) ist.

9. Zusammensetzung nach Anspruch 8, wobei das PEG ein durchschnittliches Molekulargewicht zwischen etwa 3000 und etwa 100.000 kDa aufweist.

10. Zusammensetzung nach Anspruch 8, wobei ein PEG an das rhTSH angelagert ist.

11. Zusammensetzung nach Anspruch 8, wobei mehr als ein PEG an das rhTSH angelagert ist.

12. Verfahren zur Herstellung eines biologisch aktiven rekombinanten menschlichen Thyroidea-stimulierenden Hormons (rhTSH), das mit mindestens einem Polyalkylenglykolpolymer konjugiert ist, das Verfahren umfassend ein Anbringen mindestens eines Polyalkylenglykolpolymers an ein rhTSH, an:
a) einer Sialinsäuregruppe, ausgewählt aus der Gruppe bestehend aus ASN52 der rhTSH-α-Untereinheit, ASN78 der rhTSH-α-Untereinheit und ASN23 der rhTSH-ß-Untereinheit oder einer Kombination davon; oder
b) einer Galaktose, die sich an ASN52 der rhTSH α-Untereinheit, ASN78 der rhTSH α-Untereinheit, ASN23 der rhTSH β-Untereinheit oder einer Kombination davon befindet.

13. Verfahren zur Herstellung eines biologisch aktiven mutierten rekombinanten menschlichen Thyroidea-stimulierenden Hormons (rhTSH), das mit mindestens einem Polyalkylenglykolpolymer konjugiert ist, das Verfahren umfassend
a) Einführen eines oder mehrerer zusätzlicher Cysteinreste in rhTSH, wodurch ein mutiertes rhTSH erzeugt wird, wobei der Cysteinrest einen endogenen Aminosäurerest ersetzt, der sich an der Alpha-Untereinheit von rhTSH an einer Aminosäureposition befindet, die ausgewählt ist aus der Gruppe, bestehend aus ASN66, THR69 und GLY22 und Kombinationen davon, oder der sich an der Beta-Untereinheit von rhTSH an der Aminosäureposition von VAL118 und Kombinationen davon befindet; und
b) Binden eines oder mehrerer Polyalkylenglykolpolymere an den einen oder die mehreren in Schritt a) eingeführten Cysteinreste, wodurch ein biologisch aktives mutiertes rhTSH erzeugt wird, das an mindestens ein Polyalkylenglykolpolymer konjugiert ist.

14. Verfahren nach Anspruch 13, wobei das Polyalkylenglykolpolymer Polyethylenglykol (PEG) ist.

15. Verfahren nach Anspruch 14, wobei das PEG ein durchschnittliches Molekulargewicht zwischen etwa 3000 und etwa 100.000 kDa aufweist.

16. Verfahren nach Anspruch 14, wobei ein PEG an das rhTSH angelagert ist.

17. Verfahren nach Anspruch 14, wobei mehr als ein PEG an das rhTSH angelagert ist.

18. Biologisch aktives rekombinantes menschliches Thyroidea-stimulierendes Hormon (rhTSH) zur Verwendung beim Behandleln eines Thyroidea-Zustands bei einem Patienten, der dessen bedarf, wobei mindestens ein Polyalkylenglykolpolymer an das rhTSH angelagert ist, an:
a) einer Sialinsäuregruppe, ausgewählt aus der Gruppe bestehend aus ASN52 der rhTSH-α-Untereinheit, ASN78 der rhTSH-α-Untereinheit und ASN23 der rhTSH-ß-Untereinheit oder einer Kombination davon; oder
b) einer Galaktose, die sich an ASN52 der rhTSH α-Untereinheit, ASN78 der rhTSH α-Untereinheit, ASN23 der rhTSH β-Untereinheit oder einer Kombination davon befindet.
und wobei eine wirksame Menge des biologisch aktiven rhTSH an den Patienten verabreicht wird.

19. Biologisch aktives rhTSH zur Verwendung nach Anspruch 18, wobei das Polyalkylenglykolpolymer Polyethylenglykol (PEG) ist.

20. Biologisch aktives rhTSH zur Verwendung nach Anspruch 19, wobei das PEG ein durchschnittliches Molekulargewicht zwischen etwa 3000 und etwa 100.000 kDa aufweist.

21. Biologisch aktives rhTSH zur Verwendung nach Anspruch 19, wobei ein PEG an das rhTSH angelagert ist.

22. Biologisch aktives rhTSH zur Verwendung nach Anspruch 19, wobei mehr als ein PEG an das rhTSH angelagert ist.

23. Pharmazeutische Zusammensetzung, umfassend eine Verbindung nach einem der Ansprüche 1-11 und einen pharmazeutisch annehmbaren Träger.

24. Pharmazeutische Zusammensetzung nach Anspruch 23 zur Verwendung bei der Behandlung eines Thyroidea-Zustands bei einem Patienten, der dessen bedarf, wobei dem Patienten eine wirksame Menge einer pharmazeutischen Zusammensetzung nach Anspruch 23 verabreicht wird.

25. Pharmazeutische Zusammensetzung zur Verwendung nach Anspruch 24, wobei die pharmazeutische Zusammensetzung eine verstärkte T4-Antwort im Vergleich zu einer Kontrolle aufweist.

26. Pharmazeutische Zusammensetzung zur Verwendung nach Anspruch 24, wobei der Thyroidea-Zustands Schilddrüsenkrebs ist.

27. Pharmazeutische Zusammensetzung zur Verwendung nach Anspruch 24, wobei die pharmazeutische Zusammensetzung durch intramuskuläre Injektion zugeführt wird.

## Revendications

1. Composition comprenant une thyréostimuline humaine recombinante (rhTSH), dans laquelle au moins un polymère de polyalkylène glycol est attaché à la rhTSH au niveau de :
a) un groupe acide sialique sélectionné dans le groupe constitué par ASN52 de la sous-unité rhTSH a, ASN78 de la sous-unité rhTSH a et ASN23 de la sous-unité rhTSH β ou une combinaison de celles-ci ; ou
b) un galactose situé au niveau d'ASN52 de la sous-unité rhTSH a, d'ASN78 de la sous-unité rhTSH a, d'ASN23 de la sous-unité rhTSH β ou d'une combinaison de celles-ci.

2. Composition selon la revendication 1, dans laquelle le polymère de polyalkylène glycol est le polyéthylène glycol (PEG).

3. Composition selon la revendication 10, dans laquelle le PEG a une masse moléculaire moyenne comprise entre environ 3 000 et environ 100 000 kDa.

4. Composition selon la revendication 3, dans laquelle un PEG est attaché à la rhTSH.

5. Composition selon la revendication 3, dans laquelle plus d'un PEG est attaché à la rhTSH.

6. Composition selon la revendication 1, dans laquelle la rhTSH présente une réponse T4 prolongée par rapport à un contrôle.

7. Composition comprenant une thyréostimuline humaine recombinante (rhTSH) mutée et au moins un polymère de polyalkylène glycol, dans laquelle la rhTSH mutée a un ou plusieurs résidus d'acide aminé substitués par un résidu de cystéine,
dans laquelle le résidu d'acide aminé qui a été substitué par la cystéine est situé sur la sous-unité alpha de la rhTSH à une position d'acide aminé choisie dans le groupe constitué par ASN66, THR69 et GLY22, et des combinaisons de celles-ci ; ou est situé sur la sous-unité bêta de la rhTSH à la position d'acide aminé VAL118 ; ou toute combinaison de celles-ci ; et
dans laquelle le polymère de polyalkylène glycol est fixé à la rhTSH mutée au niveau du résidu de cystéine, et la rhTSH mutée est biologiquement active.

8. Composition selon la revendication 7, dans laquelle le polymère de polyalkylène glycol est le polyéthylène glycol (PEG).

9. Composition selon la revendication 8, dans laquelle le PEG a une masse moléculaire moyenne comprise entre environ 3 000 et environ 100 000 kDa.

10. Composition selon la revendication 8, dans laquelle un PEG est attaché à la rhTSH.

11. Composition selon la revendication 8, dans laquelle plus d'un PEG est attaché à la rhTSH.

12. Procédé de production d'une thyréostimuline humaine recombinante (rhTSH) biologiquement active conjuguée à au moins un polymère de polyalkylène glycol, le procédé comprenant la fixation d'au moins un polymère de polyalkylène glycol à une rhTSH au niveau de :
a) un groupe acide sialique choisi dans le groupe constitué par ASN52 de la sous-unité rhTSH a, ASN78 de la sous-unité rhTSH a et ASN23 de la sous-unité rhTSH β ou une combinaison de celles-ci ; ou
b) un galactose situé au niveau d'ASN52 de la sous-unité rhTSH a, d'ASN78 de la sous-unité rhTSH a, d'ASN23 de la sous-unité rhTSH β ou d'une combinaison de ceux-ci.

13. Procédé de production d'une thyréostimuline humaine recombinante (rhTSH) mutée biologiquement active conjuguée à au moins un polymère de polyalkylène glycol, le procédé comprenant les étapes suivantes :
a) introduction d'un ou plusieurs résidus de cystéine supplémentaires dans la rhTSH, produisant ainsi une rhTSH mutée, dans laquelle le résidu de cystéine remplace un résidu d'acide aminé endogène situé sur la sous-unité alpha de la rhTSH à une position d'acide aminé choisie dans le groupe constitué par ASN66, THR69 et GLY22, et des combinaisons de celles-ci, ou situé sur la sous-unité bêta de la rhTSH à la position d'acide aminé VAL118, et les combinaisons de celles-ci ; et
b) fixation d'un ou plusieurs polymères de polyalkylène glycol à un ou plusieurs résidus de cystéine introduits à l'étape a), produisant ainsi une rhTSH mutée biologiquement active conjuguée à au moins un polymère de polyalkylène glycol.

14. Procédé selon la revendication 13, dans lequel le polymère de polyalkylène glycol est le polyéthylène glycol (PEG).

15. Procédé selon la revendication 14, dans lequel le PEG a une masse moléculaire moyenne comprise entre environ 3 000 et environ 100 000 kDa.

16. Procédé selon la revendication 14, dans lequel un PEG est attaché à la rhTSH.

17. Procédé selon la revendication 14, dans lequel plus d'un PEG est attaché à la rhTSH.

18. Thyréostimuline humaine recombinante (rhTSH) biologiquement active destinée à être utilisée pour le traitement d'une affection thyroïdienne chez un sujet en ayant besoin, dans laquelle au moins un polymère de polyalkylène glycol est fixé à la rhTSH au niveau de :
a) un groupe acide sialique choisi dans le groupe constitué par ASN52 de la sous-unité rhTSH a, ASN78 de la sous-unité rhTSH a et ASN23 de la sous-unité rhTSH β ou une combinaison de celles-ci ; ou
b) un galactose situé au niveau d'ASN52 de la sous-unité rhTSH a, d'ASN78 de la sous-unité rhTSH a, d'ASN23 de la sous-unité rhTSH β ou d'une combinaison de celles-ci,
et dans laquelle une quantité efficace de la rhTSH biologiquement active est administrée au patient.

19. RhTSH biologiquement active à utiliser selon la revendication 18, dans laquelle le polymère de polyalkylène glycol est le polyéthylène glycol (PEG).

20. RhTSH biologiquement active à utiliser selon la revendication 19, dans laquelle le PEG a une masse moléculaire moyenne comprise entre environ 3 000 et environ 100 000 kDa.

21. RhTSH biologiquement active à utiliser selon la revendication 19, dans laquelle un PEG est attaché à la rhTSH.

22. RhTSH biologiquement active à utiliser selon la revendication 19, dans laquelle plus d'un PEG est attaché à la rhTSH.

23. Composition pharmaceutique comprenant une quantité thérapeutique efficace d'une composition selon l'une quelconque des revendications 1 à 11 et un véhicule pharmaceutiquement acceptable.

24. Composition pharmaceutique selon la revendication 23 à utiliser dans le traitement d'une affection thyroïdienne chez un patient en ayant besoin, dans laquelle une quantité efficace d'une composition pharmaceutique selon la revendication 23 est administrée au patient.

25. Composition pharmaceutique à utiliser selon la revendication 24, dans laquelle la composition pharmaceutique présente une réponse T4 améliorée par rapport à un contrôle.

26. Composition pharmaceutique à utiliser selon la revendication 24, dans laquelle l'affection thyroïdienne est un cancer de la thyroïde.

27. Composition pharmaceutique à utiliser selon la revendication 24, dans laquelle la composition pharmaceutique est délivrée par injection intramusculaire.
